# EUROPEAN PATENT SPECIFICATION

(11) **EP 3 212 292 B1**
(45) Date of publication and mention of the grant of the patent: **20.01.2021**
(21) Application number: 15794096.6
(22) Date of filing: 27.10.2015
(51) Int. Cl.: A61Q 3/02, A61K 8/58, A61Q 19/00, A61K 8/04, C07F 7/18, C08G 18/10, C08G 18/28

(54) **POLYMER COMPRISING ALKOXYSILANE GROUPS AND USE IN COSMETICS**
POLYMER MIT ALKOXYSILANGRUPPEN UND VERWENDUNG IN DER KOSMETIK
POLYMÈRE COMPRENANT DES GROUPES ALCOXYSILANE ET UTILISATION EN COSMÉTIQUE

(30) Priority: 29.10.2014 FR 1460393
(43) Date of publication of application: 06.09.2017
(73) Proprietor: L'OREAL, 75008 Paris (FR)
(72) Inventor: GIUSTINIANI, Pascal, F-92250 La Garenne Colombes (FR)
(74) Representative: L'Oreal
(86) International application number: PCT/EP2015/074807
(87) International publication number: WO 2016/066610

(56) References cited:
- EP-A1- 1 788 035
- EP-A1- 2 725 044
- US-A1- 2002 146 382
- US-B2- 8 648 162

## Description

The present invention relates to a process for preparing a product containing polymeric compounds comprising an alkoxysilane group and to the use of such a product in a cosmetic composition for treating keratin materials, in particular the nails and the hair.

The use of sol/gel techniques for the purposes of preparing cosmetic compositions is known per se. Such compositions form films after application to keratin materials. After drying, a hybrid material is in fact formed by polycondensation and crosslinking.

For example, patent application WO 98/44906 describes a cosmetic or dermatological composition suitable for forming a coating on keratin materials via a reaction of sol/gel type obtained by mixing (A) at least one organometallic compound with (B) at least one functionalized organic polymer or at least one functionalized silicone polymer other than the first compound, and (C) an amount of water sufficient to hydrolyse the organometallic compound. US 2002/146382 A1 discloses a process for preparing a polyurethane comprising alkoxysilane groups for cosmetic applications (nails and hair), in order to prepare films with good humidity resistance while providing excellent gloss, feel and adhesion. Example 1 describes the reaction between PCPSSIP, PCL-diol, EG, DEG and IPDI in a first step. Then, the prepolymer obtained is reacted with 3-aminopropyltriethoxysilane.

There is a need to provide compounds or compositions that have both stability properties before application thereof to keratin materials, and good reactivity.

There is also a need to have compounds which make it possible to obtain, after application thereof to keratin materials, a uniform and smooth deposit.

Furthermore, the layer of the composition deposited on the keratin materials is not tacky. It thus has a good appearance and is not degraded on contact with foreign bodies, for instance a glass, a cigarette, clothing or skin.

In addition, properties of persistence on washing with water and with detergents are sought.

It is sought to obtain a film which is resistant in particular to mechanical attacks such as rubbing, and is adherent.

Finally, sheen properties of the deposited film may be sought.

The inventors have found that such advantages may be obtained by using a polymeric product comprising particular alkoxysilane groups, as described hereinafter.

After application to keratin materials, the polymeric product, on contact with the moisture in the air, crosslinks to form a film. The film obtained is shiny and has good water resistance.

The polymeric product is thus suitable for use as a film-forming agent in a nail varnish composition or a hair composition, in particular for haircare or else for hair shaping (styling), or else in a composition for caring for or making up the skin, in particular for filling the surface irregularities of the skin, such as wrinkles or grooves.

More specifically, a subject of the present invention is a process for preparing a polymer comprising alkoxysilane groups (referred to as Pf) which can be obtained by polycondensation, comprising, in a first step, the reaction between:
(i) a diisocyanate of formula (I):

   OCN-Z-NCO (I)

   in which Z denotes a divalent hydrocarbon-based radical containing from 4 to 20 carbon atoms; and
(ii) a difunctional compound of formula (II): H-T-A-T-H (II)
in which:
T denotes a heteroatom chosen from O and S or an -N(R)- radical, R being H or a C₁-C₄ alkyl radical, in particular methyl,
A denotes a linear or branched, divalent hydrocarbon-based C₂-C₁₀₀ radical, optionally interrupted with one or more non-adjacent heteroatoms chosen from O and S, or an -N(R')- group in which R' denotes a hydrogen atom or a C₁-C₄ alkyl radical, in particular methyl;
in order to form a prepolymer (P) containing at least one isocyanate function, preferably containing 2 isocyanate functions;
followed by a second step in which the prepolymer (P) obtained is reacted with a first alkoxysilane of formula (III) and a second alkoxysilane of formula (IV):

   (R₁O)(R₂)(R₃)Si-CH₂-(NH-L₁)ₚ-X₁-H (III)

   in which
   p = 0 or 1;
   X₁ denotes -NRa-, S or O, Ra denoting H or a saturated or unsaturated C₁-C₈ (cyclo)alkyl radical, in particular methyl or cyclohexyl, or a C₆-C₁₀ aryl radical, in particular phenyl;
   R₁ denotes a C₁-C₆ alkyl radical;
   R₂ and R₃, which may be identical or different, preferably identical, are chosen from:
      - a C₁-C₆, in particular C₁-C₄, alkoxy radical;
      - a linear or branched C₁-C₆ alkyl radical;
   L₁ denotes a linear or branched, saturated divalent hydrocarbon-based C₁-C₂₀ radical;

   (R'₁O)(R'₂)(R'₃)Si-CH(R₄)-CH(R₅)-(L₂)_{q}-X₂-H (IV)

   in which:
   q = 0 or 1;
   X₂ denotes -NRb- or S or O or -NHCO-NRc-, Rb denoting H or a saturated or unsaturated C₁-C₈ (cyclo)alkyl radical, such as methyl, ethyl, butyl or cyclohexyl, or a C₆-C₁₀ aryl radical such as phenyl; Rc denoting a saturated C₁-C₄ alkyl radical, in particular methyl;
   R'₁ denotes a C₁-C₆ alkyl radical;
   R'₂ and R'₃, which may be identical or different, preferably identical are chosen from:
      - a C₁-C₆, in particular C₁-C₄, alkoxy radical;
      - a linear or branched C₁-C₆ alkyl radical;
   R₅ denotes H or a C₁-C₄ alkyl radical optionally substituted with an -NH₂ group; R₄ denotes H or a C₁-C₄ alkyl radical, in particular methyl;
   L₂ denotes a linear or branched, saturated divalent hydrocarbon-based C₁-C₂₀ radical, optionally interrupted with an -NH- group, optionally substituted with an NH₂ group,

it being possible for the mixture of the first and second alkoxysilanes (III) and (IV) to be added either simultaneously or sequentially by, for example, first introducing the first alkoxysiloxane (III) then the second alkoxysilane (IV), or else by first introducing the second alkoxysilane (IV) then the first alkoxysilane (III).

Advantageously, the reagents are used in the preparation process according to the following molar equivalents:
diisocyanate (I): 2 equivalents
difunctional compound (II): 1 equivalent
first alkoxysilane (III): u equivalent
second alkoxysilane (IV): v equivalent
with u + v = 2 (u and v not being zero).

A subject of the invention is also the product (Pf) which can be obtained according to the preparation process described above.

For the diisocyanate (I) defined above, the Z radical is preferably chosen from the following radicals (1) to (6):

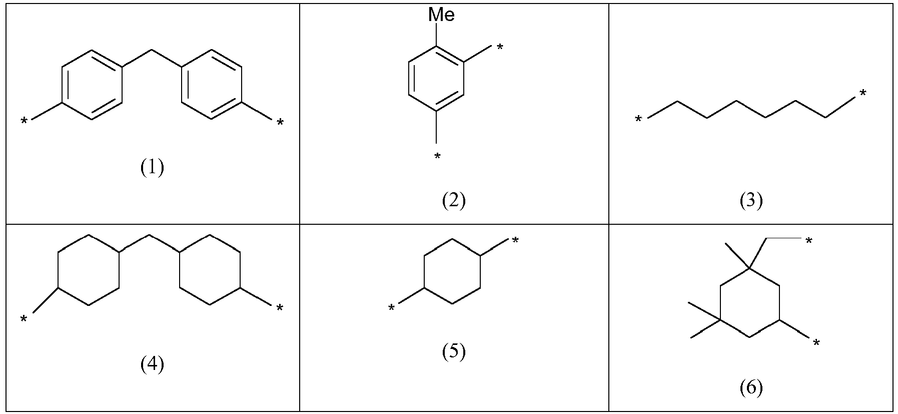

In the structures drawn, the * denotes the point of attachment with the isocyanate function. Preferentially, Z denotes the divalent radical isophorone (radical (6)).

For the difunctional compound of formula (II) defined above:
T preferably denotes O or NH;
A preferably denotes a linear or branched hydrocarbon-based C₂-C₅₀ radical optionally interrupted with one or more non-adjacent oxygen atoms.

In particular, A denotes a divalent radical chosen from:
(i) a divalent radical -[(CH₂)ₙ-O-]ₘ-(CH₂)_{n'}- with n and n' independently denoting a number between 1 and 10 (limits included), preferably between 1 and 5, m denoting a number between 1 and 30, preferably between 1 and 20, more particularly between 1 and 10;
(ii) a C₂-C₅₀, preferably C₂₀-C₅₀, in particular C₃₂-C₄₀, alkylene radical, which is in particular non-linear, such as a C₃₆ radical;
(iii) a divalent radical -[CH(Me)-CH₂-O-]ₓ-(CH₂) CH(Me)- with x denoting a number between 2 and 70 (limits included), preferably between 2 and 7; in particular, x = 2.5 or 6.1 or 33 or 68;
(iv) a divalent radical -CH(Me)-CH₂-[O-CH₂-CH(Me)]_{x'}-[O-CH₂-CH₂]_{y'}-[O-CH₂-CH(Me)-]_{z'} with y' denoting a number between 2 and 50 (limits included), preferably between 2 and 40 and in particular y' = 2 or 9 or 12.5 or 39, x' + z' denoting a number between 1 and 10 (limits included), preferably between 1 and 7; in particular, x' + z' = 1.2 or 3.6 or 6;
(v) a divalent radical -(CH₂)_{x"}-O-CH₂-CH₂-O-(CH₂)_{x"}- with x" denoting a number between 1 and 10 (limits included), preferably between 1 and 5; in particular x" = 2 or 3.

For the first alkoxysilane (III) defined above:
X₁ preferably denotes -NRa-, Ra being defined as above.

Preferentially, NRa denotes -NH- or -N-cyclohexyl.

R₁ preferably denotes a methyl or ethyl radical;

R₂ and R₃, which may be identical or different, preferably identical, are preferably chosen from:
- methoxy or ethoxy radicals;
- methyl or ethyl, preferably methyl, radicals.

Preferably, L₁ represents a linear or branched, saturated hydrocarbon-based C₁-C₁₀ radical, more particularly a saturated linear hydrocarbon-based C₁-C₁₀ radical.
According to one preferred embodiment, when p=1, L₁ represents a saturated divalent C₁-C₈ radical and in particular a divalent radical chosen from -CH₂-CH₂- and -(CH₂)₆-.

Advantageously, the first alkoxysilane (III) can be chosen from those of formula (IIIa) below:

(R₁O)(R₂)(R₃)Si-CH₂-(NH-L₁)ₚ-NRa-H (IIIa)

in which:
p = 0 or 1;
R₁ denotes a methyl or ethyl radical; R₂ and R₃, which may be identical or different, denote a methoxy, ethoxy, methyl or ethyl radical;
when p = 1, L₁ represents a saturated divalent hydrocarbon-based C₁-C₈ radical;
Ra denotes H or a saturated or unsaturated C₁-C₈ (cyclo)alkyl radical, or a phenyl radical; preferably, Ra denotes H or a cyclohexyl radical.

As examples of a first alkoxysilane (III), mention may be made of:

| Chemical name | CAS No. | Chemical structure |
|---|---|---|
| 1-(dimethoxymethylsilyl)methanamine | (343926-26-1) | 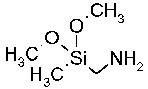 |
| 1-(diethoxymethylsilyl)methanamine | (18186-77-1) | 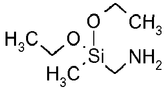 |
| 1-(triethoxysilyl)methanamine | (18306-83-7) | 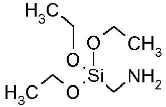 |
| 1-(trimethoxysilyl)methanamine | (71408-48-5) | 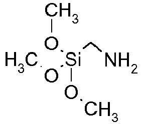 |
| 1-(trimethoxysilyl)methanethiol | (30817-94-8) | 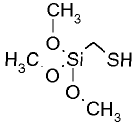 |
| 1-(diethoxymethylsilyl)methanethiol | (55161-63-2) | 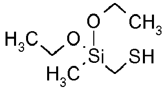 |
| 1-(triethoxysilyl)methanethiol | (60764-83-2) | 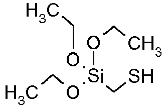 |
| 1-(triethoxysilyl)methanol | (162781-70-6) | 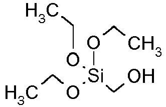 |
| N-[(triethoxysilyl)methyl]benzenamine | (3473-76-5) | 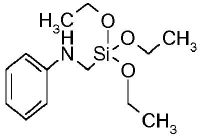 |
| N-[(trimethoxysilyl)methyl]benzenamine | (77855-73-3) | 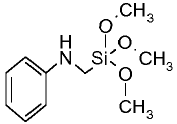 |
| N-[(diethoxymethylsilyl)methyl]cyclohexanamine | 27445-54-1 | 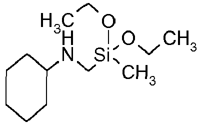 |
| N-[(triethoxysilyl)methyl]cyclohexanamine | (26495-91-0) | 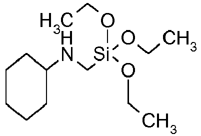 |
| N-[(dimethoxymethylsilyl)methyl]-cyclohexanamine | (733051-93-9) | 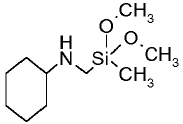 |
| N-(diethoxymethylsilyl)-N-methylmethanamine | (18306-82-6) | 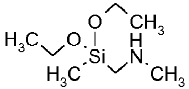 |
| N-methyl-1-(trimethoxysilyl)methanamine | (123271-16-9) | 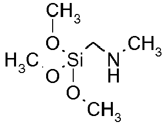 |
| N-methyl-1-(triethoxysilyl)methanamine | (151734-80-4) | 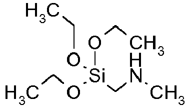 |
| N-[(dimethoxy(methyl)silyl)methyl]benzenamine | (17890-10-7) | 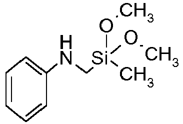 |
| N-[(triethoxysilyl)methyl]-1,6-hexanediamine | (15129-36-9) | 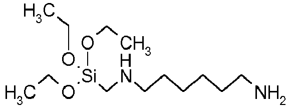 |
| N-[(trimethoxysilyl)methyl]-1,6-hexanediamine | (172684-43-4) | 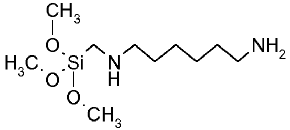 |
| N-[(diethoxymethylsilyl)methyl]-1,6-hexanediamine | (15383-20-7) | 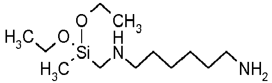 |
| N-[(trimethoxysilyl)methyl]-1,2-ethanediamine | (51980-40-6) | 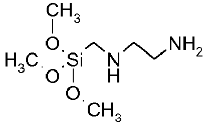 |

As examples of a preferred first alkoxysilane (III), mention may be made of:
1-(diethoxymethylsilyl)methanamine (18186-77-1)
1-(triethoxysilyl)methanamine (18306-83-7)
N-[(diethoxymethylsilyl)methyl]cyclohexanamine (27445-54-1)
N-[(triethoxysilyl)methyl]cyclohexanamine (26495-91-0)
N-[(triethoxysilyl)methyl]-1,6-hexanediamine (15129-36-9)

More preferentially, use is made, as first alkoxysilane (III), of
N-[(triethoxysilyl)methyl]-1,6-hexanediamine (15129-36-9)
N-[(triethoxysilyl)methyl]cyclohexanamine (26495-91-0).

For the second alkoxysilane (IV) defined above:
X₂ preferably denotes -NRb- or S, Rb denoting H or a saturated or unsaturated C₁-C₈ (cyclo)alkyl radical, such as methyl, ethyl, butyl or cyclohexyl, or a C₆-C₁₀ aryl radical such as phenyl. Preferentially, X₂ denotes -NH- or S.

Preferably, L₂ represents a linear or branched, saturated hydrocarbon-based C₁-C₁₂ radical, optionally interrupted with an -NH- group.
More preferentially, when q = 1, L₂ represents a saturated divalent C₁-C₁₀ radical, or else a divalent radical -(CH₂)ₙ-NH-(CH₂)ₘ with n and m denoting integers such that 2 ≤ n + m ≤ 4,
and in particular a divalent radical chosen from -CH₂-, -CH₂-CH₂-, -(CH₃)(CH₃)C-CH₂-, -(CH₂)₉- and -CH₂-NH-C₂H₄-.

R'₁ preferably denotes a methyl or ethyl radical;

R'₂ and R'₃, which may be identical or different, preferably identical, are preferably chosen from:
- methoxy or ethoxy radicals;
- methyl or ethyl, preferably methyl, radicals.

R'₄ and R'₅ preferably denote H.

Advantageously, the second alkoxysilane (IV) can be chosen from those of formula (IVa) below:

(R'₁O)(R'₂)(R₃)Si-CH₂-CH₂-(L₂)_{q}-NRb-H (IVa)

in which:
q = 0 or 1;
Rb denoting H or a saturated or unsaturated C₁-C₈ (cyclo)alkyl radical, such as methyl, ethyl, butyl or cyclohexyl, or a phenyl radical;
R'₁ denotes a methyl or ethyl radical; R'₂ and R'₃, which may be identical or different, denote a methoxy, ethoxy, methyl or ethyl radical;
L₂ denotes a linear or branched, saturated hydrocarbon-based C₁-C₁₂ radical, optionally interrupted with an -NH- group.

More preferentially, when q = 1, L₂ represents a saturated divalent C₁-C₁₀ radical, or else a divalent radical -(CH₂)ₙ-NH-(CH₂)ₘ with n and m denoting integers such that 2 ≤ n +m ≤ 4, and in particular a divalent radical chosen from -CH₂-, -CH₂-CH₂-, -(CH₃)(CH₃)C-CH₂-, -(CH₂)₉- and -CH₂-NH-C₂H₄-; and more particularly a divalent radical chosen from -CH₂-, -CH₂-CH₂-, -(CH₃)(CH₃)C-CH₂- and -(CH₂)₉-.

As examples of a second alkoxysilane (IV), mention may be made of:

| Chemical name | CAS No. | Chemical structure |
|---|---|---|
| 3-(dimethoxymethylsilyl)-1-propanamine | (3663-44-3) | 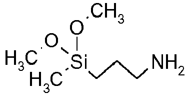 |
| 3-(trimethoxysilyl)-1-propanamine | (13822-56-5) | 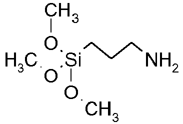 |
| 3-(triethoxysilyl)-1-propanamine | (919-30-2) | 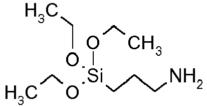 |
| 3-(diethoxymethoxysilyl)-1-propanamine | (61083-96-3) | 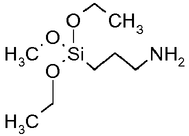 |
| 2-methyl-3-(trimethoxysilyl)-1-propanamine | (99503-87-4) | 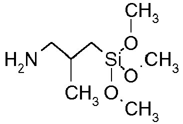 |
| 3-(triethoxysilyl)-1-propanamine | (29159-37-3) | 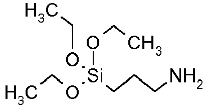 |
| 3-(diethoxymethylsilyl)-1-propanamine | (3179-76-8) | 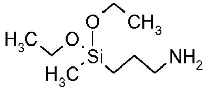 |
| 3-(methyldipropoxysilyl)-1-propanamine | (55081-09-9) | 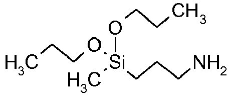 |
| 3-(diethoxyethylsilyl)-1-propanamine | (20723-29-9) | 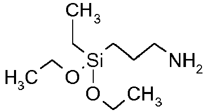 |
| 3-(ethyldimethoxysilyl)-1-propanamine | (639464-90-7) | 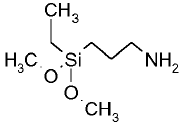 |
| 4-(triethoxysilyl)-1-butanamine | (3069-30-5) | 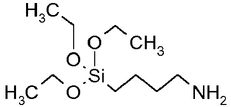 |
| 4-(dimethoxymethylsilyl)-1-butanamine | (18306-81-5) | 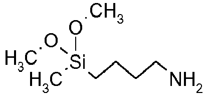 |
| 4-(trimethoxysilyl)-1-butanamine | (15005-59-1) | 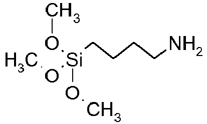 |
| 2,2-dimethyl-4-(trimethoxysilyl)-1-butanamine | (157923-74-5) | 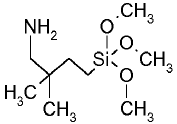 |
| 4-(diethoxymethylsilyl)-1-butanamine | (3037-72-7) | 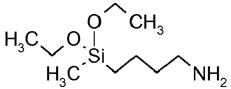 |
| 4-(dimethoxymethylsilyl)-2,2-dimethyl-1-butanamine | (156849-43-3) | 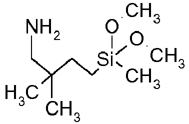 |
| 11-(triethoxysilyl)-1-undecamine | (116821-45-5) | 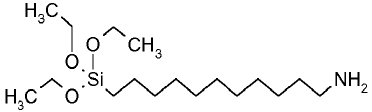 |
| 11-(trimethoxysilyl)-1-undecamine | (40762-31-0) | 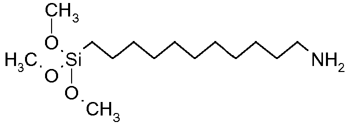 |
| 2-[(dimethoxymethylsilyl)methyl]-1,4-butanediamine | (1019109-96-6) | 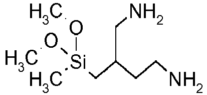 |
| 2-[(trimethoxysilyl)methyl]-1,4-butanediamine | (6037-49-6) | 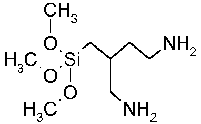 |
| N-(3-(trimethoxysilyl)propyl)butylamine | (31024-56-3) | 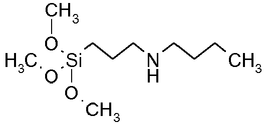 |
| N-ethyl-3-(trimethoxysilyl)-1-propanamine | (3451-81-8) | 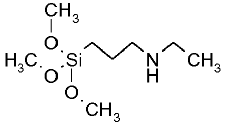 |
| N-methyl-3-(trimethoxysilyl)propylamine | (3069-25-8) | 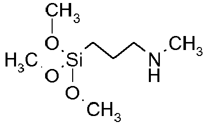 |
| N-[3-trimethoxysilyl]propyl]cyclohexylamine | (3068-78-8) | 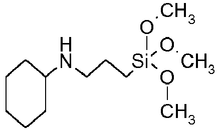 |
| N-[3-tri methoxysilyl]propyl]aniline | (3068-76-6) | 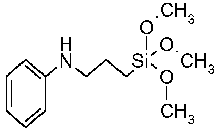 |
| N-[3-trimethoxysilyl]propyl]ethylenediamine | (1760-24-3) | 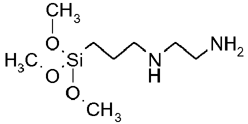 |
| N-[3-triethoxysilyl]propyl]ethylenediamine | (5089-72-5) | 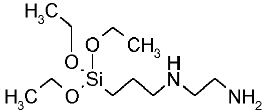 |
| 1-(trimethoxysilyl)-2-propanamine | (130530-83-5) | 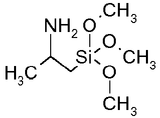 |
| 2-(trimethoxysilyl)ethanamine | (65644-31-7) | 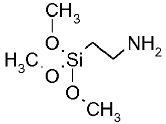 |
| 2-(triethoxysilyl)-1-propanamine | (36957-84-3) | 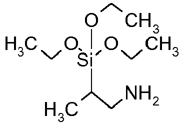 |
| 2-(dimethoxymethylsilyl)ethanamine | (115599-33-2) | 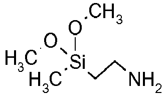 |
| 2-(diethoxymethylsilyl)-1-propanamine | (53813-15-3) | 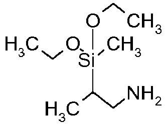 |
| 2-(diethoxymethylsilyl)ethanamine | (51250-43-2) | 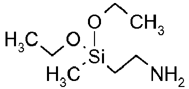 |
| 2-(triethoxysilyl)ethanamine | (45074-31-5) | 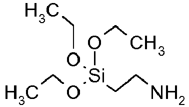 |
| 4-(trimethoxysilyl)-1-butanol | (177072-52-5) | 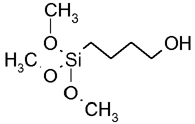 |
| 3-(trimethoxysilyl)-1-propanol | (53764-54-8) | 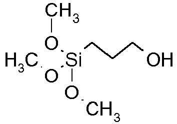 |
| 11-(trimethoxysilyl)-1-undecanethiol | (877593-17-4) | 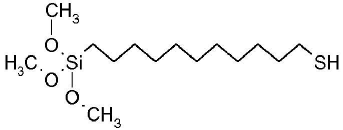 |
| 4-(trimethoxysilyl)-2-butanethiol | (57640-10-5) | 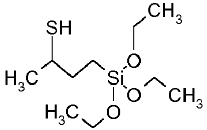 |
| 2-(triethoxysilyl)ethanethiol | (18236-15-2) | 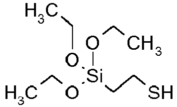 |
| 3-(triethoxysilyl)-1-propanethiol | (14814-09-6) | 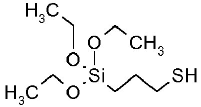 |
| 2-(trimethoxysilyl)ethanethiol | (7538-45-6) | 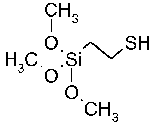 |
| 3-(trimethoxysilyl)-1-propanethiol | (4420-74-0) | 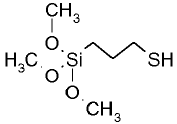 |
| 3-(dimethoxymethylsilyl)-1-propanethiol | (31001-77-1) | 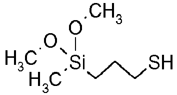 |
| N-[3-(trimethoxysilyl)propyl]acetamide | (57757-66-1) | 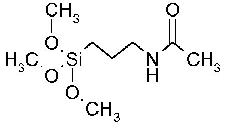 |

As examples of a preferred second alkoxysilane (IV), mention may be made of:
3-(triethoxysilyl)-1-propanamine (919-30-2)
3-(diethoxymethoxysilyl)-1-propanamine (61083-96-3)
3-(triethoxysilyl)-1-propanamine (29159-37-3)
3-(diethoxymethylsilyl)-1-propanamine (3179-76-8)
3-(diethoxyethylsilyl)-1-propanamine (20723-29-9)
4-(triethoxysilyl)-1-butanamine (3069-30-5)
4-(diethoxymethylsilyl)-1-butanamine (3037-72-7)
11-(triethoxysilyl)-1-undecamine (116821-45-5)
N-[3-triethoxysilyl]propyl]ethylenediamine (5089-72-5)
2-(triethoxysilyl)ethanethiol (18236-15-2)
3-(triethoxysilyl)-1-propanethiol (14814-09-6)

As particularly preferred alkoxysilane (IV), mention may be made of (3-aminopropyl)triethoxysilane and 3-(triethoxysilyl)-1-propanethiol.

In the process according to the invention, the first and second alkoxysilanes (III) and (IV) can be used in any relative proportions.
Preferably, the mixture of alkoxysilane (III) and (IV) used comprises from 5 to 95 mol% of alkoxysilane (III), relative to the total moles of alkoxysilanes (III) and (IV).
Preferentially, the mixture of alkoxysilane (III) and (IV) used comprises from 20 to 80 mol% of alkoxysilane (III), relative to the total moles of alkoxysilanes (III) and (IV).
In particular, the mixture of alkoxysilane (III) and (IV) used comprises from 30 to 70 mol% of alkoxysilane (III), relative to the total moles of alkoxysilanes (III) and (IV).
In particular, the mixture of alkoxysilane (III) and (IV) used comprises from 50 to 70 mol% of alkoxysilane (III), relative to the total moles of alkoxysilanes (III) and (IV).

It is understood that the amount of alkoxysilane (IV) in these mixtures is the remainder to 100 mol% adding to the molar amount of alkoxysilane (III) indicated.

As indicated above, in the second step of the process, the alkoxysilanes (III) and (IV) can be added simultaneously to the prepolymer (P) or else added sequentially by, for example, first adding the alkoxysilane (III) then the alkoxysilane (IV), or else first the alkoxysilane (IV) then the alkoxysilane (III).

In the preparation process described above, the first step can be carried out in the presence of a catalyst, in particular a tin-based organic catalyst, such as tin 2-ethylhexanoate, dibutyltin dilaurate, dioctyltin dilaurate, butyltin tris(2-ethylhexanoate), dibutyltin diacetate or dioctyltin diacetate, and preferably tin 2-ethylhexanoate.

Advantageously, the first step of the preparation process is carried out in an aprotic solvent, such as methyltetrahydrofuran, tetrahydrofuran or toluene,
at a temperature of between 40°C and 120°C, in particular between 50°C and 70°C.
The first step may be carried out with a reaction time ranging from 3 to 7 hours.

The second step of the preparation process may be carried out at a temperature of between 20°C and 60°C, in particular at ambient temperature (25°C). This second step may be carried out with a reaction time ranging from 2 to 12 hours.

After the second step of the process, it is possible to carry out a solvent exchange, according to the techniques known to those skilled in the art, in particular by elimination of the aprotic solvent (gradual elimination by distillation) and addition of a carrier solvent of the obtained polymer comprising an alkoxysilane group (Pf). The carrier solvent may be an alcohol solvent, in particular a C₂-C₂₂ alcohol solvent, such as ethanol, isopropanol, propanol, t-butanol, sec-butanol or 2-octyldodecanol.

Advantageously, the obtained polymer comprising an alkoxysilane group (Pf) is carried in a carrier solvent, in particular an alcohol solvent as described above.

The preparation process described above can be represented schematically according to the following simplified reaction scheme.

### First step:

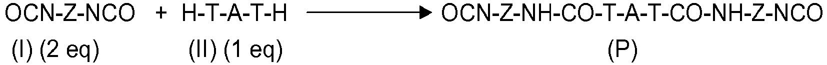

### Second step:

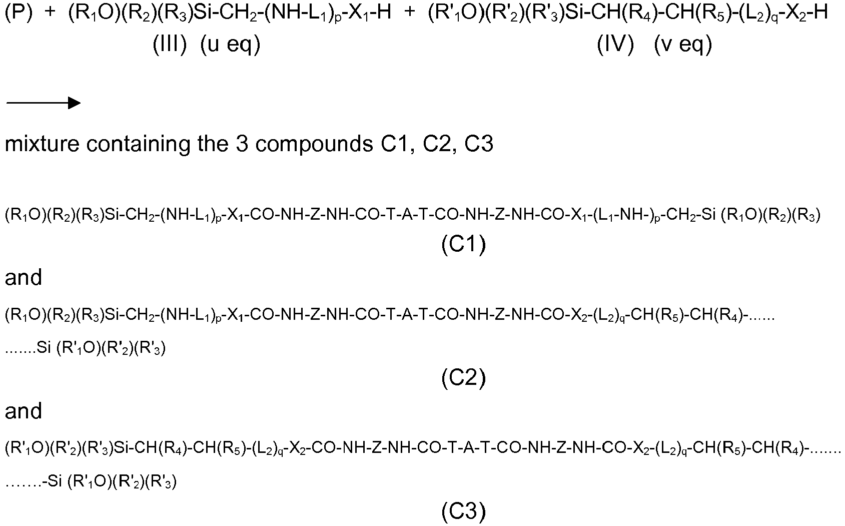

u denoting the number of molar equivalents of alkoxysilane (III) placed in reaction v denoting the number of molar equivalents of alkoxysilane (IV) placed in reaction
with u + v = 2, v other than 0.
Preferably, u is greater than 1.

According to one preferred embodiment of the preparation process, u is between 0.1 and 1.9 (limits included). Preferably, u is between 0.4 and 1.6. Preferentially, u is between 0.6 and 1.4. In particular, u is between 1 and 1.4.

The final product is obtained at the end of the reaction (total consumption of the isocyanate functions) in the form of a solution in a solvent which may be the reaction solvent or a carrier solvent, such as an alcohol solvent, in particular by solvent exchange, as described above.

The simplified reaction scheme described above is an illustration of the case corresponding to the formation of the pure prepolymer (P). Nevertheless, the prepolymer (P) can be obtained as a mixture with other compounds resulting from the condensation of (I) with (II) and/or (II) with (P); thus, it is possible to obtain, in the final product (Pf), other compounds which are additional to the compounds C1, C2 and C3, resulting in particular from the polycondensation of the compound (P) with the compound (II), then of these products with the compounds (III) and/or (IV).

A subject of the invention is the product which is a polymer comprising an alkoxysilane group (Pf), which can be obtained with the preparation process described above.

As indicated above, the preparation process makes it possible to obtain a mixture comprising the compounds C1, C2 and C3 described above.
Thus, a subject of the invention is the mixture of the compounds C1, C2 and C3.
A further subject of the invention is the compound C2 as novel compound and for which T denotes O or NH; A denotes a linear or branched hydrocarbon-based C₂-C₅₀ radical optionally interrupted with one or more non-adjacent oxygen atoms.

A further subject of the invention is the compound C3 as novel compound.

A further subject of the invention is an anhydrous composition comprising, in a physiologically acceptable medium, a product or compound or mixture of compounds as defined above. The term "physiologically acceptable medium" is intended to mean a medium that is compatible with keratin materials such as the skin, the hair or the nails, as a cosmetic medium.

In particular, the composition comprises the product (Pf) obtained according to the preparation process described above.

In particular, the composition comprises a mixture of the compounds C1, C2 and C3 as described above.

The product (Pf) or the mixture of compounds comprising C1, C2, C3 may be present in the composition according to the invention in a content ranging from 0.1% to 60% by weight, relative to the total weight of the composition, preferably ranging from 0.1% to 50% by weight, preferentially ranging from 0.5% to 45% by weight.

A further subject of the invention is a process, in particular a cosmetic process, for caring for or making up keratin materials, in particular the nails or the hair or the skin, comprising the application to the keratin materials, in particular to the nails or the hair or the skin, of a composition as described above.

According to one particular embodiment, the composition according to the present invention may also comprise at least one volatile organic solvent.
The term "volatile organic solvent" denotes, in the present invention, an organic compound which is liquid at ambient temperature (25°C), which comprises at least one group chosen from hydroxyl, ester, ketone, ether or aldehyde groups, and which has a vapour pressure greater than 1 mbar (100 Pa) at 20°C.

Among the volatile organic solvents that may be used in the composition in accordance with the invention, mention may be made of lower monoalcohols containing from 1 to 5 carbon atoms, such as ethanol and isopropanol, C₃-C₄ ketones, C₂-C₄ aldehydes and C₂-C₄ short-chain esters.

The composition according to the invention is anhydrous. The term "anhydrous" is intended to mean a composition comprising a content of less than or equal to 2% and in particular 1% by weight of water, relative to the total weight of the composition, or is even free of water. It is in particular intended to mean that water is preferably not deliberately added to the composition, but may be present in trace amounts in the various compounds used in the composition.

The composition according to the invention may also comprise a cosmetic adjuvant chosen from film-forming polymers, plasticizers, colorants, preservatives, fragrances, fillers, oils, waxes, thickeners, antioxidants, surfactants and skin care active agents.

The invention will now be described with reference to the examples that follow, which are given as non-limiting illustrations.

### Example 1:

22.2 g (0.10 mol) of isophorone diisocyanate, 100 µl of tin 2-ethylhexanoate catalyst and 270 g of methyltetrahydrofuran (MeTHF) (dried over sieve) are introduced into a 500 ml reactor equipped with a dropping funnel, under an argon atmosphere. The solution was heated to 55°C. 26.7 g (0.05 mol) of C₃₆ non-linear diol dimer (Pripol ® 2033 from Croda) diluted in 20 g of MeTHF were then added over the course of 40 minutes. At the end of the addition, heating was carried out at 65°C until half the isocyanate functions had been consumed.
20.5 g (0.07 mol) of N-(6-aminohexyl)aminomethyltriethoxysilane in 30 g of MeTHF were introduced into the dropping funnel and then this mixture was added to the reactor at ambient temperature, over the course of 30 min. 6.6 g (0.03 mol) of (3-aminopropyl) triethoxysilane diluted in 10 g of MeTHF were then added dropwise. Reaction was allowed to take place for 4 hours and then 200 g of isopropanol were added and the resulting mixture was heated for 1 hour at 60°C; the temperature was then increased in order to distill off the MeTHF. When half the amount of MeTHF had been distilled off, 100 g of isopropanol were added and the distillation was continued. The operation was repeated until the MeTHF had been totally removed. In the end, a solution containing a mixture of compounds (Pf) with a solids content of 60% by weight in isopropanol was obtained.

The solution obtained contains the following compounds: 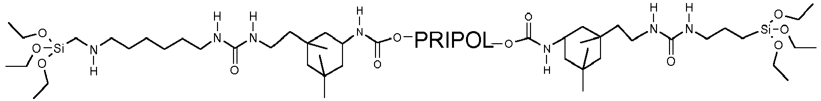 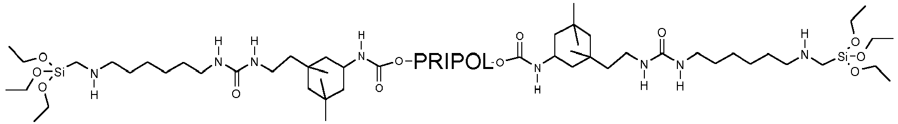 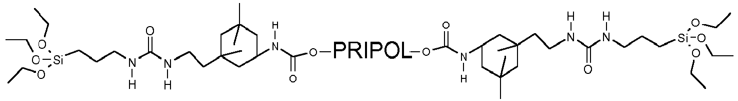 with Pripol denoting the non-linear divalent C₃₆ radical of the dimer diol Pripol 2033.

The solution obtained, comprising the mixture of these compounds, applied to a Teflon® plate, rapidly forms a film. The film obtained is uniform, transparent, shiny and non-tacky. The film obtained was subsequently detached from the plate and then placed in a crystallizer filled with water and with stirring for 24 hours at 25°C: after this time, it was noted that the film remains in a state that is still transparent and shiny and therefore has good water resistance

### Example 2:

A nail varnish having the following composition (as weight percentage) is prepared:

| | |
|---|---|
| solution obtained according to Example 1 | 99% |
| Red 27 pigment | 1% |

The varnish composition, after application to false nails, forms, on contact with the air, a uniform, glossy and water- and scratch-resistant film.

### Example 3:

A hair composition as follows, packaged in a pump dispenser bottle, is prepared:

| | | |
|---|---|---|
| solution obtained according to Example 1 | | 1%AM |
| ethanol | qs | 100% |

After the application of the composition to the hair, the latter is shiny and also has more body (it is not lank). It is easier to style.

### Example 4:

A skincare composition as follows, packaged in a pump dispenser bottle, is prepared:

| | | |
|---|---|---|
| solution obtained according to Example 1 | | 3% AM |
| 2-octyldodecanol | qs | 100% |

A few drops of the composition are deposited on the finger and the product is then applied to the wrinkled area of the face. After application, the deposit formed fills the relief of the treated skin, and the area treated appears smoother.

## Claims

1. Process for preparing a polymer comprising alkoxysilane groups which can be obtained by polycondensation, comprising, in a first step, the reaction between:
(i) a diisocyanate of formula (I):
OCN-Z-NCO (I)
in which Z denotes a divalent hydrocarbon-based radical containing from 4 to 20 carbon atoms;
and
(ii) a difunctional compound of formula (II):
H-T-A-T-H (II)
in which:
T denotes a heteroatom chosen from O and S or an -N(R)- radical, R being H or a C₁-C₄ alkyl radical, in particular methyl,
A denotes a linear or branched, divalent hydrocarbon-based C₂-C₁₀₀ radical, optionally interrupted with one or more non-adjacent heteroatoms chosen from O and S, or an -N(R')- group in which R' denotes a hydrogen atom or a C₁-C₄ alkyl radical, in particular methyl;
in order to form a prepolymer (P) containing at least one isocyanate function, preferably containing 2 isocyanate functions;
followed by a second step in which the prepolymer (P) obtained is reacted with a first alkoxysilane of formula (III) and a second alkoxysilane of formula (IV):
(R₁O)(R₂)(R₃)Si-CH₂-(NH-L₁)ₚ-X₁-H (III)
in which
p = 0 or 1;
X₁ denotes -NRa-, S or O, Ra denoting H or a saturated or unsaturated C₁-C₈ (cyclo)alkyl radical, in particular methyl or cyclohexyl, or a C₆-C₁₀ aryl radical, in particular phenyl;
R₁ denotes a C₁-C₆ alkyl radical;
R₂ and R₃, which may be identical or different, preferably identical are chosen from:
- a C₁-C₆, in particular C₁-C₄, alkoxy radical;
- a linear or branched C₁-C₆ alkyl radical;
L₁ denotes a linear or branched, saturated divalent hydrocarbon-based C₁-C₂₀ radical;
(R'₁O)(R'₂)(R'₃)Si-CH(R₄)-CH(R₅)-(L₂)_{q}-X₂-H (IV)
in which:
q = 0 or 1;
X₂ denotes -NRb- or S or O or -NHCO-NRc-, Rb denoting H or a saturated or unsaturated C₁-C₈ (cyclo)alkyl radical, in particular a methyl, ethyl, butyl or cyclohexyl radical, or a C₆-C₁₀ aryl radical, in particular phenyl; Rc denoting a saturated C₁-C₄ alkyl radical, in particular methyl;
R'₁ denotes a C₁-C₆ alkyl radical;
R'₂ and R'₃, which may be identical or different, are chosen from:
- a C₁-C₆, in particular C₁-C₄, alkoxy radical;
- a linear or branched C₁-C₆ alkyl radical;
R₄ denotes H or a C₁-C₄ alkyl radical, in particular methyl;
R₅ denotes H or a C₁-C₄ alkyl radical optionally substituted with an -NH₂ group;
L₂ denotes a linear or branched, saturated divalent hydrocarbon-based C₁-C₂₀ radical, optionally interrupted with an -NH- group, optionally substituted with an NH₂ group;
it being possible for the first and second alkoxysilanes (III) and (IV) to be added either simultaneously or sequentially by first introducing the first alkoxysiloxane (III) then the second alkoxysilane (IV), or by first introducing the second alkoxysilane (IV) then the first alkoxysilane (III).

2. Process according to the preceding claim, **characterized in that**, for the diisocyanate (I), the Z radical is chosen from the following radicals (1) to (6): 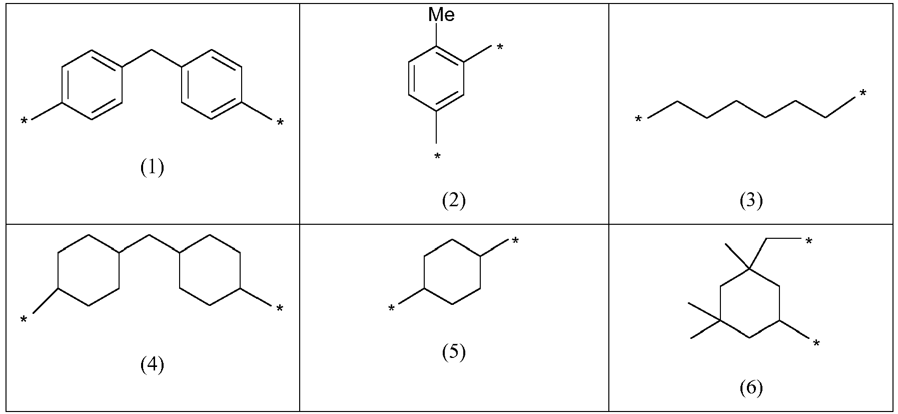 preferably Z denotes the divalent radical isophorone (radical (6)).

3. Process according to either of the preceding claims, **characterized in that**, for the difunctional compound of formula (II):
T denotes O or NH;
A denotes a linear or branched hydrocarbon-based C₂-C₅₀ radical optionally interrupted with one or more non-adjacent oxygen atoms;
preferably, A denotes a divalent radical chosen from:
(i) a divalent radical -[(CH₂)ₙ-O-]ₘ-(CH₂)_{n'}- with n and n' independently denoting a number between 1 and 10, preferably between 1 and 5, m denoting a number between 1 and 30, preferably between 1 and 20, more particularly between 1 and 10;
(ii) a C₂-C₅₀, preferably C₂₀-C₅₀, in particular C₃₂-C₄₀, alkylene radical, which is in particular non-linear, such as a C₃₆ radical;
(iii) a divalent radical -[CH(Me)-CH₂-O-]ₓ-(CH₂) CH(Me)- with x denoting a number between 2 and 70, preferably between 2 and 7;
(iv) a divalent radical -CH(Me)-CH₂-[O-CH₂-CH(Me)]_{x'}-[O-CH₂-CH₂]_{y'}-[O-CH₂-CH(Me)-]_{z'} with y' denoting a number between 2 and 50, preferably 2 and 40, x' + z' denoting a number between 1 and 10, preferably between 1 and 7;
(v) a divalent radical -(CH₂)_{x"}-O-CH₂-CH₂-O-(CH₂)_{x"}- with x" denoting a number between 1 and 10, preferably between 1 and 5.

4. Process according to one of the preceding claims, **characterized in that**, for the first alkoxysilane (III):
X₁ denotes -NRa-, preferably -NRa- denotes -NH- or -N-cyclohexyl;
- R₁ denotes a methyl or ethyl radical;
- R₂ and R₃, which may be identical or different, are chosen from:
- methoxy or ethoxy radicals;
- methyl or ethyl, preferably methyl, radicals;
L₁ represents a linear or branched, saturated hydrocarbon-based C₁-C₁₀ radical, preferably a saturated linear hydrocarbon-based C₁-C₁₀ radical; preferably, when p=1, L₁ represents a saturated divalent C₁-C₈ radical, preferably a divalent radical chosen from -CH₂-CH₂- and -(CH₂)₆-.

5. Process according to one of the preceding claims, **characterized in that** the first alkoxysilane (III) is chosen from those of formula (IIIa) below:
(R₁O)(R₂)(R₃)Si-CH₂-(NH-L₁)ₚ-NRa-H (IIIa)
in which:
p = 0 or 1;
R₁ denotes a methyl or ethyl radical; R₂ and R₃, which may be identical or different, denote a methoxy, ethoxy, methyl or ethyl radical;
when p = 1, L₁ represents a saturated divalent hydrocarbon-based C₁-C₈ radical;
Ra denotes H or a saturated or unsaturated C₁-C₈ (cyclo)alkyl radical, or a phenyl radical; preferably, Ra denotes H or a cyclohexyl radical.

6. Process according to one of the preceding claims, **characterized in that** the first alkoxysilane (III) is chosen from:
1-(dimethoxymethylsilyl)methanamine
1-(diethoxymethylsilyl)methanamine
1-(triethoxysilyl)methanamine
1-(trimethoxysilyl)methanamine
1-(trimethoxysilyl)methanethiol
1-(diethoxymethylsilyl)methanethiol
1-(triethoxysilyl)methanethiol
1-(triethoxysilyl)methanol
N-[(triethoxysilyl)methyl]benzenamine
N-[(trimethoxysilyl)methyl]benzenamine
N-[(diethoxymethylsilyl)methyl]cyclohexanamine
N-[(triethoxysilyl)methyl]cyclohexanamine
N-[(dimethoxymethylsilyl)methyl]-cyclohexanamine
N-(diethoxymethylsilyl)-N-methylmethanamine
N-methyl-1-(trimethoxysilyl)methanamine
N-methyl-1-(triethoxysilyl)methanamine
N-[(dimethoxy(methyl)silyl)methyl]benzenamine
N-[(triethoxysilyl)methyl]-1,6-hexanediamine
N-[(trimethoxysilyl)methyl]-1,6-hexanediamine
N-[(diethoxymethylsilyl)methyl]-1,6-hexanediamine
N-[(trimethoxysilyl)methyl]-1,2-ethanediamine,
preferably from:
1-(diethoxymethylsilyl)methanamine
1-(triethoxysilyl)methanamine
N-[(diethoxymethylsilyl)methyl]cyclohexanamine
N-[(triethoxysilyl)methyl]cyclohexanamine
N-[(triethoxysilyl)methyl]-1,6-hexanediamine,
and preferentially from:
N-[(triethoxysilyl)methyl]-1,6-hexanediamine
N-[(triethoxysilyl)methyl]cyclohexanamine.

7. Process according to one of the preceding claims, **characterized in that**, for the second alkoxysilane (IV):
X₂ denotes -NRb- or S, Rb denoting H or a saturated or unsaturated C₁-C₈ (cyclo)alkyl radical, in particular a methyl, ethyl, butyl or cyclohexyl radical, or a C₆-C₁₀ aryl radical, in particular phenyl; preferably, X₂ denotes -NH- or S;
L₂ represents a linear or branched, saturated hydrocarbon-based C₁-C₁₂ radical, optionally interrupted with an -NH- group; preferably, when q= 1, L₂ represents a saturated divalent C₁-C₁₀ radical, or else a divalent radical -(CH₂)ₙ-NH-(CH₂)ₘ with n and m denoting integers such that 2 ≤ n + m ≤ 4, and in particular a divalent radical chosen from -CH₂-, -CH₂-CH₂-, -(CH₃)(CH₃)C-CH₂-, -(CH₂)₉- and -CH₂-NH-C₂H₄-;
R'₁ denotes a methyl or ethyl radical,
R'₂ and R'₃, which may be identical or different, are chosen from:
- methoxy or ethoxy radicals;
- methyl or ethyl, preferably methyl, radicals;
R'₄ and R'₅ denote H.

8. Process according to one of the preceding claims, **characterized in that** the second alkoxysilane (IV) is chosen from those of formula (IVa) below:
(R'₁O)(R'₂)(R₃)Si-CH₂-CH₂-(L₂)_{q}-NRb-H (IVa)
in which:
q = 0 or 1;
Rb denoting H or a saturated or unsaturated C₁-C₈ (cyclo)alkyl radical, such as methyl, ethyl, butyl or cyclohexyl, or a phenyl radical;
R'₁ denotes a methyl or ethyl radical; R'₂ and R'₃, which may be identical or different, denote a methoxy, ethoxy, methyl or ethyl radical;
L₂ denotes a linear or branched, saturated hydrocarbon-based C₁-C₁₂ radical, optionally interrupted with an -NH- group; preferably, when q= 1, L₂ represents a saturated divalent C₁-C₁₀ radical, or else a divalent radical -(CH₂)ₙ-NH-(CH₂)ₘ with n and m denoting integers such that 2 ≤ n + m ≤ 4, and in particular a divalent radical chosen from -CH₂-, -CH₂-CH₂-, -(CH₃)(CH₃)C-CH₂-, -(CH₂)₉- and -CH₂-NH-C₂H₄-.

9. Process according to one of the preceding claims, **characterized in that** the second alkoxysilane (IV) is chosen from:
3-(dimethoxymethylsilyl)-1-propanamine
3-(trimethoxysilyl)-1-propanamine
3-(triethoxysilyl)-1-propanamine
3-(diethoxymethoxysilyl)-1-propanamine
2-methyl-3-(trimethoxysilyl)-1-propanamine
3-(triethoxysilyl)-1-propanamine
3-(diethoxymethylsilyl)-1-propanamine
3-(methyldipropoxysilyl)-1-propanamine
3-(diethoxyethylsilyl)-1-propanamine
3-(ethyldimethoxysilyl)-1-propanamine
4-(triethoxysilyl)-1-butanamine
4-(dimethoxymethylsilyl)-1-butanamine
4-(trimethoxysilyl)-1-butanamine
2,2-dimethyl-4-(trimethoxysilyl)-1-butanamine
4-(diethoxymethylsilyl)-1-butanamine
4-(dimethoxymethylsilyl)-2,2-dimethyl-1-butanamine
11-(triethoxysilyl)-1-undecamine
11-(trimethoxysilyl)-1-undecamine
2-[(dimethoxymethylsilyl)methyl]-1,4-butanediamine
2-[(trimethoxysilyl)methyl]-1,4-butanediamine
N-(3-(trimethoxysilyl)propyl)butylamine
N-ethyl-3-(trimethoxysilyl)-1-propanamine
N-methyl-3-(trimethoxysilyl)propylamine
N-[3-trimethoxysilyl]propyl]cyclohexylamine
N-[3-trimethoxysilyl]propyl]aniline
N-[3-trimethoxysilyl]propyl]ethylenediamine
N-[3-triethoxysilyl]propyl]ethylenediamine
1-(trimethoxysilyl)-2-propanamine
2-(trimethoxysilyl)ethanamine
2-(triethoxysilyl)-1-propanamine
2-(dimethoxymethylsilyl)ethanamine
2-(diethoxymethylsilyl)-1-propanamine
2-(diethoxymethylsilyl)ethanamine
2-(triethoxysilyl)ethanamine
4-(trimethoxysilyl)-1-butanol
3-(trimethoxysilyl)-1-propanol
11-(trimethoxysilyl)-1-undecanethiol
4-(trimethoxysilyl)-2-butanethiol
2-(triethoxysilyl)ethanethiol
3-(triethoxysilyl)-1-propanethiol
2-(trimethoxysilyl)ethanethiol
3-(trimethoxysilyl)-1-propanethiol
3-(dimethoxymethylsilyl)-1-propanethiol
N-[3-(trimethoxysilyl)propyl]acetamide,
preferably from:
3-(triethoxysilyl)-1-propanamine
3-(diethoxymethoxysilyl)-1-propanamine
3-(triethoxysilyl)-1-propanamine
3-(diethoxymethylsilyl)-1-propanamine
3-(diethoxyethylsilyl)-1-propanamine
4-(triethoxysilyl)-1-butanamine
4-(diethoxymethylsilyl)-1-butanamine
11-(triethoxysilyl)-1-undecamine
N-[3-triethoxysilyl]propyl]ethylenediamine
2-(triethoxysilyl)ethanethiol
3-(triethoxysilyl)-1-propanethiol,
and preferentially chosen from (3-aminopropyl) triethoxysilane and 3-(triethoxysilyl)-1-propanethiol.

10. Process according to one of the preceding claims, **characterized in that** the mixture of first and second alkoxysilanes (III) and (IV) used comprises from 5 to 95 mol% of alkoxysilane (III), relative to the total moles of alkoxysilanes (III) and (IV), preferably from 20 to 80 mol%, preferentially from 30 to 70 mol%, and more preferentially from 50 to 70 mol%.

11. Process according to one of the preceding claims, **characterized in that** the reagents are used according to the following molar equivalents:
diisocyanate (I): 2 equivalents
difunctional compound (II): 1 equivalent
first alkoxysilane (III): u equivalent
second alkoxysilane (IV): v equivalent
with u + v = 2, u and v not being zero.

12. Process according to one of the preceding claims, **characterized in that** the first step is carried out in the presence of a catalyst, in particular a tin-based organic catalyst, such as tin 2-ethylhexanoate, dibutyltin dilaurate, dioctyltin dilaurate, butyltin tris(2-ethylhexanoate), dibutyltin diacetate or dioctyltin diacetate, and preferably tin 2-ethylhexanoate.

13. Process according to one of the preceding claims, **characterized in that** the first step is carried out in an aprotic solvent, preferably chosen from methyltetrahydrofuran, tetrahydrofuran and toluene, at a temperature of between 40°C and 120°C, preferably between 50°C and 70°C.

14. Process according to one of the preceding claims, **characterized in that** the second step is carried out at a temperature of between 20°C and 60°C.

15. Process according to one of the preceding claims, **characterized in that** the second step is followed by a step of solvent exchange by elimination of the aprotic solvent and addition of a carrier solvent, such as an alcohol solvent, in particular a C₂-C₂₂ alcohol solvent, preferably chosen from ethanol, isopropanol, propanol, t-butanol, sec-butanol and 2-octyldodecanol.

16. Process according to one of the preceding claims, **characterized in that** the obtained polymer comprising an alkoxysilane group is carried in a carrier solvent, in particular an alcohol solvent as defined in the preceding claim.

17. Product which is a polymer comprising an alkoxysilane group (Pf), which is obtained with the preparation process according to any one of the preceding claims.

18. Mixture of compounds C1, C2 and C3:
(R₁O)(R₂)(R₃)Si-CH₂-(NH-L₁)ₚ-X₁-CO-NH-Z-NH-CO-T-A-T-CO-NH-Z-NH-CO-X₁-(L₁-NH-)ₚ-CH₂-Si(R₁O)(R₂)(R₃) (C1)
(R₁O)(R₂)(R₃)Si-CH₂-(NH-L₁)ₚ-X₁-CO-NH-Z-NH-CO-T-A-T-CO-NH-Z-NH-CO-X₂-(L₂)_{q}-CH(R₅)-CH(R₄)-...... .......Si (R'₁O)(R'₂)(R'₃) (C2)
(R'₁O)(R'₂)(R'₃)Si-CH(R₄)-CH(R₅)-(L₂)_{q}-X₂-CO-NH-Z-NH-CO-T-A-T-CO-NH-Z-NH-CO-X₂-(L₂)_{q}-CH(R₅)-CH(R₄)- ....... .......-Si(R'₁O)(R'₂)(R'₃) (C3)
in which Z, T, A, R₁, R₂, R₃, R'₁, R'₂, R'₃, R₄, R₅, L₁, L₂, X₁, X₂, p and q have the meanings defined in Claims 1 to 4 and 6 and 7.

19. Compounds of formula C2:
(R₁O)(R₂)(R₃)Si-CH₂-(NH-L₁)ₚ-X₁-CO-NH-Z-NH-CO-T-A-T-CO-NH-Z-NH-CO-X₂-(L₂)_{q}-CH(R₅)-CH(R₄)-...... .......Si (R'₁O)(R'₂)(R'₃) (C2)
in which Z, R₁, R₂, R₃, R'₁, R'₂, R'₃, R₄, R₅, L₁, L₂, X₁, X₂, p and q have the meanings defined in Claims 1 to 4 and 6 and 7, and
T denotes O or NH;
A denotes a linear or branched hydrocarbon-based C₂-C₅₀ radical optionally interrupted with one or more non-adjacent oxygen atoms.

20. Anhydrous composition comprising, in a physiologically acceptable medium, a product or compound(s) as defined according to one of Claims 17 to 19.

21. Composition according to the preceding claim, **characterized in that** the product or the compounds are present in a content ranging from 0.1% to 60% by weight, relative to the total weight of the composition, preferably ranging from 0.1% to 50% by weight, preferentially ranging from 0.5% to 45% by weight.

22. Composition according to Claim 20 or 21, **characterized in that** it comprises at least one volatile organic solvent.

23. Cosmetic process for caring for or making up keratin materials, in particular the nails or the hair or the skin, comprising the application to the keratin materials, in particular to the nails or the hair or the skin, of a composition according to either of Claims 21 and 22.

## Patentansprüche

1. Verfahren zur Herstellung eines Polymers mit Alkoxysilangruppen, das durch Polykondensation erhältlich ist, die in einem ersten Schritt die Reaktion zwischen
(i) einem Diisocyanat der Formel (I):
OCN-Z-NCO (I),
in der Z für einen zweiwertigen Rest auf Kohlenwasserstoffbasis mit 4 bis 20 Kohlenstoffatomen steht; und
(ii) einer difunktionellen Verbindung der Formel (II):
H-T-A-T-H (II),
in der:
T für ein Heteroatom, das aus O und S ausgewählt ist, oder einen -N(R)-Rest steht, wobei R H oder ein C₁-C₄-Alkylrest, insbesondere Methyl, ist,
A für einen linearen oder verzweigten, zweiwertigen C₂-C₁₀₀-Rest auf Kohlenwasserstoffbasis, der gegebenenfalls durch ein oder mehrere nicht benachbarte Heteroatome unterbrochen ist, die aus O und S ausgewählt sind, oder eine -N(R')-Gruppe, in der R' für ein Wasserstoffatom oder einen C₁-C₄-Alkylrest, insbesondere Methyl, steht, steht;
zur Bildung eines Prepolymers (P) mit mindestens einer Isocyanatfunktion, vorzugsweise mit 2 Isocyanatfunktionen;
gefolgt von einem zweiten Schritt, in dem das erhaltene Prepolymer (P) mit einem ersten Alkoxysilan der Formel (III) und einem zweiten Alkoxysilan der Formel (IV) umgesetzt wird:
(R₁O) (R₂) (R₃) Si-CH₂- (NH-L₁)ₚ-X₁-H (III)
in der
p = 0 oder 1;
X₁ für -NRa-, S oder O steht, wobei Ra für H oder einen gesättigten oder ungesättigten C₁-C₈-(Cyclo)-alkylrest, insbesondere Methyl oder Cyclohexyl, oder einen C₆-C₁₀-Arylrest, insbesondere Phenyl, steht;
R₁ für einen C₁-C₆-Alkylrest steht;
R₂ und R₃, die gleich oder verschieden sein können und vorzugsweise gleich sind, aus
- einem C₁-C₆-Alkoxyrest, insbesondere einem C₁-C₄-Alkoxyrest und
- einen linearen oder verzweigten C₁-C₆-Alkylrest ausgewählt sind;
L₁ für einen linearen oder verzweigten, gesättigten zweiwertigen C₁-C₂₀-Rest auf Kohlenwasserstoffbasis steht;
(R'₁O) (R'₂) (R'₃) Si-CH(R₄) -CH(R₅) - (L₂)_{q}-X₂-H (IV)
in der:
q = 0 oder 1;
X₂ für -NRb- oder S oder O oder -NHCO-NRc- steht, wobei Rb für H oder einen gesättigten oder ungesättigten C₁-C₈-(Cyclo)alkylrest, insbesondere einen Methyl-, Ethyl-, Butyl- oder Cyclohexylrest, oder einen C₆-C₁₀-Arylrest, insbesondere Phenyl, steht; Rc für einen gesättigten C₁-C₄-Alkylrest, insbesondere Methyl, steht;
R'₁ für einen C₁-C₆-Alkylrest steht;
R'₂ und R'₃, die gleich oder verschieden sein können, aus
- einem C₁-C₆-Alkoxyrest, insbesondere einem C₁-C₄-Alkoxyrest und
- einem linearen oder verzweigten C₁-C₆-Alkylrest ausgewählt sind;
R₄ für H oder einen C₁-C₄-Alkylrest, insbesondere Methyl, steht;
R₅ für H oder einen C₁-C₄-Alkylrest, der gegebenenfalls durch eine -NH₂-Gruppe substituiert ist, steht;
L₂ für einen linearen oder verzweigten, gesättigten zweiwertigen C₁-C₂₀-Rest auf Kohlenwasserstoffbasis, der gegebenenfalls durch eine -NH-Gruppe unterbrochen und gegebenenfalls durch eine NH₂-Gruppe substituiert ist, steht;
umfasst;
wobei das erste und das zweite Alkoxysilan (III) und (IV) entweder gleichzeitig oder nacheinander, indem man zuerst das erste Alkoxysiloxan (III) und dann das zweite Alkoxysilan (IV) einträgt oder indem man zuerst das zweite Alkoxysilan (IV) und dann das erste Alkoxysilan (III) einträgt, zugegeben werden können.

2. Verfahren nach dem vorhergehenden Anspruch, **dadurch gekennzeichnet, dass** für das Diisocyanat (I) der Z-Rest aus den folgenden Resten (1) bis (6) ausgewählt ist: 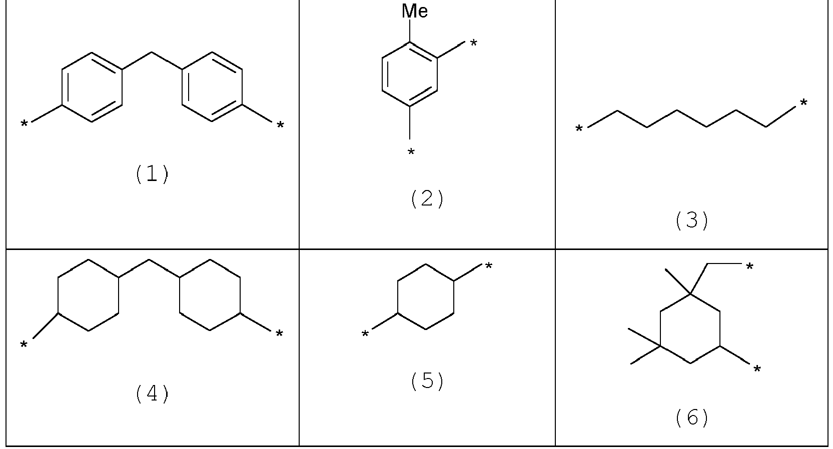 vorzugsweise Z für den zweiwertigen Rest Isophoron (Rest (6)) steht.

3. Verfahren nach einem der beiden vorhergehenden Ansprüche, **dadurch gekennzeichnet, dass** für die difunktionelle Verbindung der Formel (II):
T für O oder NH steht;
A für einen linearen oder verzweigten C₂-C₅₀-Rest auf Kohlenwasserstoffbasis, der gegebenenfalls durch ein oder mehrere nicht benachbarte Sauerstoffatome unterbrochen ist, steht;
vorzugsweise A für einen zweiwertigen Rest steht,
der aus:
(i) einem zweiwertigen Rest -[(CH₂)ₙ-O-]ₘ-(CH₂)_{n'}-, wobei n und n' unabhängig für eine Zahl zwischen 1 und 10, vorzugsweise zwischen 1 und 5, steht und m für eine Zahl zwischen 1 und 30, vorzugsweise zwischen 1 und 20, spezieller zwischen 1 und 10, steht;
(ii) einem C₂-C₅₀-, vorzugsweise C₂₀-C₅₀- und insbesondere C₃₂-C₄₀-Alkylenrest, der insbesondere nicht linear ist, wie ein C₃₆-Rest;
(iii) einem zweiwertigen Rest -[CH(Me)-CH₂-O-]ₓ-(CH₂)-CH(Me)-, wobei x für eine Zahl zwischen 2 und 70, vorzugsweise zwischen 2 und 7, steht;
(iv) einem zweiwertigen Rest -CH(Me)-CH₂-[O-CH₂-CH(Me)]_{x'}-[O-CH₂-CH₂]_{y'}-[O-CH₂-CH(Me)-]_{z'}, wobei y' für eine Zahl von 2 und 50, vorzugsweise zwischen 2 und 40, steht und x' + z' für eine Zahl zwischen 1 und 10, vorzugsweise zwischen 1 und 7, steht;
(v) einem zweiwertigen Rest -(CH₂)_{x"}-O-CH₂-CH₂-O-(CH₂)_{x"}-, wobei x'' für eine Zahl zwischen 1 und 10, vorzugsweise zwischen 1 und 5, steht;
ausgewählt ist.

4. Verfahren nach einem der vorhergehenden Ansprüche, **dadurch gekennzeichnet, dass** für das erste Alkoxysilan (III):
X₁ für -NRa- steht, vorzugsweise -NRa- für -NH- oder -N-Cyclohexyl steht;
- R₁ für einen Methyl- oder Ethylrest steht;
- R₂ und R₃, die gleich oder verschieden sein können, aus:
- Methoxy- oder Ethoxyresten;
- Methyl- oder Ethylresten, vorzugsweise Methylresten;
ausgewählt sind;
- L₁ für einen linearen oder verzweigten, gesättigten C₁-C₁₀-Rest auf Kohlenwasserstoffbasis, vorzugsweise einen gesättigten linearen C₁-C₁₀-Rest auf Kohlenwasserstoffbasis, steht; vorzugsweise im Fall von p = 1 L₁ für einen gesättigten zweiwertigen C₁-C₈-Rest, vorzugsweise einen zweiwertigen Rest, der aus -CH₂-CH₂- und -(CH₂)₆-ausgewählt ist, steht.

5. Verfahren nach einem der vorhergehenden Ansprüche, **dadurch gekennzeichnet, dass** das erste Alkoxysilan (III) aus denjenigen der nachstehenden Formel (IIIa) ausgewählt ist:
(R₁O) (R₂) (R₃)Si-CH₂-(NH-L₁)ₚ-NRa-H (IIIa)
in der:
p = 0 oder 1;
R₁ für einen Methyl- oder Ethylrest steht; R₂ und
R₃, die gleich oder verschieden sein können, für einen Methoxy-, Ethoxy-, Methyl- oder Ethylrest stehen;
im Fall von p = 1 L₁ für einen gesättigten zweiwertigen C₁-C₈-Rest auf Kohlenwasserstoffbasis steht;
Ra für H oder einen gesättigten oder ungesättigten C₁-C₈-(Cyclo)alkylrest oder einen Phenylrest steht;
vorzugsweise Ra für H oder einen Cyclohexylrest steht.

6. Verfahren nach einem der vorhergehenden Ansprüche, **dadurch gekennzeichnet, dass** das erste Alkoxysilan (III) aus:
1-(Dimethoxymethylsilyl)methanamin
1-(Diethoxymethylsilyl)methanamin
1-(Triethoxysilyl)methanamin
1-(Trimethoxysilyl)methanamin
1-(Trimethoxysilyl)methanthiol
1-(Diethoxymethylsilyl)methanthiol
1-(Triethoxysilyl)methanthiol
1-(Triethoxysilyl)methanol
N-[(Triethoxysilyl)methyl]benzolamin
N-[(Trimethoxysilyl)methyl]benzolamin
N-[(Diethoxymethylsilyl)methyl]cyclohexanamin
N-[(Triethoxysilyl)methyl]cyclohexanamin
N-[(Dimethoxymethylsilyl)methyl]cyclohexanamin
N-(Diethoxymethylsilyl)-N-methylmethanamin
N-Methyl-1-(trimethoxysilyl)methanamin
N-Methyl-1-(triethoxysilyl)methanamin
N-[(Dimethoxy(methyl)silyl)methyl]benzolamin
N-[(Triethoxysilyl)methyl]-1,6-hexandiamin
N-[(Trimethoxysilyl)methyl]-1,6-hexandiamin
N-[(Diethoxymethylsilyl)methyl]-1,6-hexandiamin
N-[(Trimethoxysilyl)methyl]-1,2-ethandiamin
vorzugsweise aus:
1-(Diethoxymethylsilyl)methanamin
1-(Triethoxysilyl)methanamin
N-[(Diethoxymethylsilyl)methyl]cyclohexanamin
N-[(Triethoxysilyl)methyl]cyclohexanamin
N-[(Triethoxysilyl)methyl]-1,6-hexandiamin,
und bevorzugt aus:
N-[(Triethoxysilyl)methyl]-1,6-hexandiamin
N-[(Triethoxysilyl)methyl]cyclohexanamin ausgewählt ist.

7. Verfahren nach einem der vorhergehenden Ansprüche, **dadurch gekennzeichnet, dass** für das zweite Alkoxysilan (IV):
X₂ für -NRb- oder S steht, wobei Rb für H oder einen gesättigten oder ungesättigten C₁-C₈-(Cyclo)alkylrest, insbesondere einen Methyl-, Ethyl-, Butyl- oder Cyclohexylrest, oder einen C₆-C₁₀-Arylrest, insbesondere Phenyl, steht; vorzugsweise X₂ für -NH- oder S steht;
L₂ für einen linearen oder verzweigten, gesättigten C₁-C₁₂-Rest auf Kohlenwasserstoffbasis, der gegebenenfalls durch eine -NH-Gruppe unterbrochen ist; vorzugsweise im Fall von q = 1 L₂ für einen gesättigten zweiwertigen C₁-C₁₀-Rest oder auch einen zweiwertigen Rest - (CH₂)ₙ-NH-(CH₂)ₘ, wobei n und m für solche ganzen Zahlen stehen, dass 2 ≤ n + m ≤ 4, und insbesondere einen zweiwertigen Rest, der aus -CH₂-, -CH₂-CH₂-, -(CH₃)(CH₃)C-CH₂-, -(CH₂)₉- und -CH₂-NH-C₂H₄- ausgewählt ist, steht;
R'₁ für einen Methyl- oder Ethylrest steht,
R'₂ und R'₃, die gleich oder verschieden sein können, aus:
- Methoxy- oder Ethoxyresten;
- Methyl- oder Ethylresten, vorzugsweise Methylresten;
ausgewählt sind;
R'₄ und R'₅ für H stehen.

8. Verfahren nach einem der vorhergehenden Ansprüche, **dadurch gekennzeichnet, dass** das zweite Alkoxysilan (IV) aus denjenigen der nachstehenden Formel (IVa) ausgewählt ist:
(R'₁O)(R'₂)(R₃)Si-CH₂-CH₂-(L₂)_{q}-NRb-H (IVa)
in der:
q = 0 oder 1;
wobei Rb für H oder einen gesättigten oder ungesättigten C₁-C₈-(Cyclo)alkylrest, wie Methyl, Ethyl, Butyl oder Cyclohexyl, oder einen Phenylrest steht;
R'₁ für einen Methyl- oder Ethylrest steht; R'₂ und R'₃, die gleich oder verschieden sein können, für einen Methoxy-, Ethoxy-, Methyl- oder Ethylrest stehen;
L₂ für einen linearen oder verzweigten, gesättigten C₁-C₁₂-Rest auf Kohlenwasserstoffbasis, der gegebenenfalls durch eine -NH-Gruppe unterbrochen ist; vorzugsweise im Fall von q = 1 L₂ für einen gesättigten zweiwertigen C₁-C₁₀-Rest oder auch einen zweiwertigen Rest - (CH₂)ₙ-NH-(CH₂)ₘ, wobei n und m für solche ganzen Zahlen stehen, dass 2 ≤ n + m ≤ 4, und insbesondere einen zweiwertigen Rest, der aus -CH₂-, -CH₂-CH₂-, - (CH₃)(CH₃)C-CH₂-, - (CH₂)₉- und -CH₂-NH-C₂H₄- ausgewählt ist, steht.

9. Verfahren nach einem der vorhergehenden Ansprüche, **dadurch gekennzeichnet, dass** das zweite Alkoxysilan (IV) aus:
3-(Dimethoxymethylsilyl)-1-propanamin
3-(Trimethoxysilyl)-1-propanamin
3-(Triethoxysilyl)-1-propanamin
3-(Diethoxymethoxysilyl)-1-propanamin
2-Methyl-3-(trimethoxysilyl)-1-propanamin
3-(Triethoxysilyl)-1-propanamin
3-(Diethoxymethylsilyl)-1-propanamin
3-(Methyldipropoxysilyl)-1-propanamin
3-(Diethoxyethylsilyl)-1-propanamin
3-(Ethyldimethoxysilyl)-1-propanamin
4-(Triethoxysilyl)-1-butanamin
4-(Dimethoxymethylsilyl)-1-butanamin
4-(Trimethoxysilyl)-1-butanamin
2,2-Dimethyl-4-(trimethoxysilyl)-1-butanamin
4-(Diethoxymethylsilyl)-1-butanamin
4-(Dimethoxymethylsilyl)-2,2-dimethyl-1-butanamin
11-(Triethoxysilyl)-1-undecamin
11-(Trimethoxysilyl)-1-undecamin
2-[(Dimethoxymethylsilyl)methyl]-1,4-butandiamin
2-[(Trimethoxysilyl)methyl]-1,4-butandiamin
N-(3-(Trimethoxysilyl)propyl)butylamin
N-Ethyl-3-(trimethoxysilyl)-1-propanamin
N-Methyl-3-(trimethoxysilyl)propylamin
N-[3-Trimethoxysilyl]propyl]cyclohexylamin
N-[3-Trimethoxysilyl]propyl]anilin
N-[3-Trimethoxysilyl]propyl]ethylendiamin
N-[3-Triethoxysilyl]propyl]ethylendiamin
1-(Trimethoxysilyl)-2-propanamin
2-(Trimethoxysilyl)ethanamin
2-(Triethoxysilyl)-1-propanamin
2-(Dimethoxymethylsilyl)ethanamin
2-(Diethoxymethylsilyl)-1-propanamin
2-(Diethoxymethylsilyl)ethanamin
2-(Triethoxysilyl)ethanamin
4-(Trimethoxysilyl)-1-butanol
3-(Trimethoxysilyl)-1-propanol
11-(Trimethoxysilyl)-1-undecanthiol
4-(Trimethoxysilyl)-2-butanthiol
2-(Triethoxysilyl)ethanthiol
3-(Triethoxysilyl)-1-propanthiol
2-(Trimethoxysilyl)ethanthiol
3-(Trimethoxysilyl)-1-propanthiol
3-(Dimethoxymethylsilyl)-1-propanthiol
N-[3-(Trimethoxysilyl)propyl]acetamid,
vorzugsweise aus:
3-(Triethoxysilyl)-1-propanamin
3-(Diethoxymethoxysilyl)-1-propanamin
3-(Triethoxysilyl)-1-propanamin
3-(Diethoxymethylsilyl)-1-propanamin
3-(Diethoxyethylsilyl)-1-propanamin
4-(Triethoxysilyl)-1-butanamin
4-(Diethoxymethylsilyl)-1-butanamin
11-(Triethoxysilyl)-1-undecamin
N-[3-Triethoxysilyl]propyl]ethylendiamin
2-(Triethoxysilyl)ethanthiol
3-(Triethoxysilyl)-1-propanthiol
und bevorzugt aus (3-Aminopropyl)triethoxysilan und 3-(Triethoxysilyl)-1-propanthiol
ausgewählt ist.

10. Verfahren nach einem der vorhergehenden Ansprüche, **dadurch gekennzeichnet, dass** die verwendete Mischung des ersten und zweiten Alkoxysilans (III) und (IV) 5 bis 95 Mol-% Alkoxysilan (III), bezogen auf die gesamten Mole der Alkoxysilane (III) und (IV), vorzugsweise 20 bis 80 Mol-%, bevorzugt 30 bis 70 Mol-% und weiter bevorzugt 50 bis 70 Mol-% umfasst.

11. Verfahren nach einem der vorhergehenden Ansprüche, **dadurch gekennzeichnet, dass** die Reagenzien gemäß den folgenden molaren Äquivalenten verwendet werden:
Diisocyanat (I): 2 Äquivalente
difunktionelle Verbindung (II): 1 Äquivalent
erstes Alkoxysilan (III): u Äquivalent
zweites Alkoxysilan (IV): v Äquivalent,
wobei u + v = 2, wobei u und v nicht gleich null sind.

12. Verfahren nach einem der vorhergehenden Ansprüche, **dadurch gekennzeichnet, dass** der erste Schritt in Gegenwart eines Katalysators, insbesondere eines auf Zinn basierenden organischen Katalysators, wie Zinn-2-ethylhexanoat, Dibutylzinndilaurat, Dioctylzinndilaurat, Butylzinntris(2-ethylhexanoat), Dibutylzinndiacetat oder Dioctylzinndiacetat und vorzugsweise Zinn-2-ethylhexanoat, durchgeführt wird.

13. Verfahren nach einem der vorhergehenden Ansprüche, **dadurch gekennzeichnet, dass** der erste Schritt in einem aprotischen Lösungsmittel, das vorzugsweise aus Methyltetrahydrofuran, Tetrahydrofuran und Toluol ausgewählt wird, bei einer Temperatur zwischen 40 °C und 120 °C, vorzugsweise zwischen 50 °C und 70 °C, durchgeführt wird.

14. Verfahren nach einem der vorhergehenden Ansprüche, **dadurch gekennzeichnet, dass** der zweite Schritt bei einer Temperatur zwischen 20 °C und 60 °C durchgeführt wird.

15. Verfahren nach einem der vorhergehenden Ansprüche, **dadurch gekennzeichnet, dass** auf den zweiten Schritt ein Schritt des Lösungsmittelaustauschs durch Eliminierung des aprotischen Lösungsmittels und Zugabe eines Trägerlösungsmittels, wie eines Alkohol-Lösungsmittels, insbesondere eines C₂-C₂₂-Alkohol-Lösungsmittels, das vorzugsweise aus Ethanol, Isopropanol, n-Propanol, t-Butanol, sec-Butanol und 2-Octyldodecanol ausgewählt wird, folgt.

16. Verfahren nach einem der vorhergehenden Ansprüche, **dadurch gekennzeichnet, dass** das erhaltene Polymer mit einer Alkoxysilangruppe in einem Trägerlösungsmittel, insbesondere einem Alkohol-Lösungsmittel gemäß dem vorhergehenden Anspruch, geträgert ist.

17. Produkt, bei dem es sich um ein Polymer mit einer Alkoxysilangruppe (Pf) handelt, das mit dem Herstellungsverfahren gemäß einem der vorhergehenden Ansprüche erhalten wird.

18. Gemisch von Verbindungen C1, C2 und C3:
(R₁O)(R₂)(R₃)Si-CH_{z}-(NH-L₁)ₚ,-X₁-CO-NH-Z-NH-CO-T-A-T-CO-NH-Z-NH-CO-X₁-(L₁-NH-)ₚ-CH₂-Si(R₁O)(R₂)(R₃) (C1)
(R₁O)(R₂)(R₃)Si-CH₂-(NH-L₁)ₚ-X₁-CO-NH-Z-NH-CO-T-A-T-CO-NH-Z-NH-CO-X₂-(L₂)_{q}-CH(R₅)-CH(R₄)- ... .......Si (R'₁O)(R'₂)(R'₃) (C2)
(R'₁O)(R'₂)(R'₃)Si-CH(R₄)-CH(R₅)-(L₂)_{q}-X₂-CO-NH-Z-NH-CO-T-A-T-CO-NH-Z-NH-CO-X₂-(L₂)_{q}-CH(R₅)-CH(R₄)- ....... .......Si (R'₁O)(R'₂)(R'₃) (C3)
in denen Z, T, A, R₁, R₂, R₃, R'₁, R'₂, R'₃, R₄, R₅, L₁, L₂, X₁, X₂, p und q die in den Ansprüchen 1 bis 4 und 6 und 7 definierten Bedeutungen haben.

19. Verbindungen der Formel C2:
(R₁O)(R₂)(R₃)Si-CH₂-(NH-L₁)ₚ-X₁-CO-NH-Z-NH-CO-T-A-T-CO-NH-Z-NH-CO-X₂-(L₂)_{q}-CH(R₅)-CH(R₁)-...... .......Si (R'₁O)(R'₂)(R'₃) (C2)
in der Z, R₁, R₂, R₃, R'₁, R'₂, R'₃, R₄, R₅, L₁, L₂, X₁, X₂, p und q die in den Ansprüchen 1 bis 4 und 6 und 7 definierten Bedeutungen haben und
T für O oder NH steht;
A für einen linearen oder verzweigten C₂-C₅₀-Rest auf Kohlenwasserstoffbasis, der gegebenenfalls durch ein oder mehrere nicht benachbarte Sauerstoffatome unterbrochen ist, steht.

20. Wasserfreie Zusammensetzung, die in einem physiologisch unbedenklichen Medium ein Produkt oder eine oder mehrere Verbindungen gemäß einem der Ansprüche 17 bis 19 umfasst.

21. Zusammensetzung nach dem vorhergehenden Anspruch, **dadurch gekennzeichnet, dass** das Produkt bzw. die Verbindungen in einem Gehalt im Bereich von 0,1 bis 60 Gew.-%, bezogen auf das Gesamtgewicht der Zusammensetzung, vorzugsweise im Bereich von 0,1 bis 50 Gew.-%, bevorzugt im Bereich von 0,5 bis 45 Gew.-%, vorliegen.

22. Zusammensetzung nach Anspruch 20 oder 21, **dadurch gekennzeichnet, dass** sie mindestens ein flüchtiges organisches Lösungsmittel enthält.

23. Kosmetisches Verfahren zum Pflegen oder Schminken von Keratinmaterialien, insbesondere der Nägel oder des Haars oder der Haut, bei dem man auf die Keratinmaterialien, insbesondere auf die Nägel oder das Haar oder die Haut, eine Zusammensetzung nach einem der Ansprüche 21 und 22 aufbringt.

## Revendications

1. Procédé pour la préparation d'un polymère comprenant des groupes alcoxysilane qui peut être obtenu par polycondensation, comprenant, dans une première étape, la réaction entre :
(i) un diisocyanate de formule (I) :
OCN-Z-NCO (I)
dans laquelle Z désigne un radical hydrocarboné divalent contenant de 4 à 20 atomes de carbone ; et
(ii) un composé difonctionnel de formule (II) :
H-T-A-T-H (II)
dans laquelle :
T désigne un hétéroatome choisi parmi O et S ou un radical -N(R)-, R étant H ou un radical alkyle en C₁-C₄, en particulier méthyle,
A désigne un radical hydrocarboné divalent linéaire ou ramifié en C₂-C₁₀₀, éventuellement interrompu par un ou plusieurs hétéroatomes non adjacents choisis parmi O et S, ou par un groupe -N(R')- dans lequel R' désigne un atome d'hydrogène ou un radical alkyle en C₁-C₄, en particulier méthyle ;
afin de former un prépolymère (P) contenant au moins une fonction isocyanate, de préférence contenant 2 fonctions isocyanate ;
suivie par une deuxième étape dans laquelle le prépolymère (P) obtenu est mis à réagir avec un premier alcoxysilane de formule (III) et un deuxième alcoxysilane de formule (IV) :
(R₁O)(R₂)(R₃)Si-CH₂-(NH-L₁)ₚ-X₁-H (III)
dans laquelle
p = 0 ou 1 ;
X₁ désigne -NRa-, S ou O, Ra désignant H ou un radical (cyclo)alkyle saturé ou insaturé en C₁-C₈, en particulier méthyle ou cyclohexyle, ou un radical aryle en C₆-C₁₀, en particulier phényle ;
R₁ désigne un radical alkyle en C₁-C₆ ;
R₂ et R₃, qui peuvent être identiques ou différents, préférablement identiques, sont choisis parmi :
- un radical alcoxy en C₁-C₆, en particulier en C₁-C₄ ;
- un radical alkyle linéaire ou ramifié en C₁-C₆ ;
L₁ désigne un radical hydrocarboné divalent saturé, linéaire ou ramifié, en C₁-C₂₀ ;
(R'₁O)(R'₂)(R'₃)Si-CH(R₄)-CH(R₅)-(L₂)_{q}-X₂-H (IV)
dans laquelle :
q = 0 ou 1 ;
X₂ désigne -NRb- ou S ou O ou -NHCO-NRc-, Rb désignant H ou un radical (cyclo)alkyle saturé ou insaturé en C₁-C₈, en particulier un radical méthyle, éthyle, butyle ou cyclohexyle, ou un radical aryle en C₆-C₁₀, en particulier phényle ; Rc désignant un radical alkyle en C₁-C₄, en particulier méthyle ;
R'₁ désigne un radical alkyle en C₁-C₆ ;
R'₂ et R'₃, qui peuvent être identiques ou différents, sont choisis parmi :
- un radical alcoxy en C₁-C₆, en particulier en C₁-C₄ ;
- un radical alkyle linéaire ou ramifié en C₁-C₆ ;
R₄ désigne H ou un radical alkyle en C₁-C₄, en particulier méthyle ;
R₅ désigne H ou un radical alkyle en C₁-C₄ éventuellement substitué par un groupe - NH₂;
L₂ désigne un radical hydrocarboné divalent saturé, linéaire ou ramifié, en C₁-C₂₀, éventuellement interrompu par un groupe-NH-, éventuellement substitué par un groupe NH₂ ;
dans lequel il est possible pour les premier et deuxième alcoxysilanes (III) et (IV) d'être ajoutés soit simultanément, soit séquentiellement en introduisant d'abord le premier alcoxysilane (III), puis le deuxième alcoxysilane (IV), ou en introduisant d'abord le deuxième alcoxysilane (IV), puis le premier alcoxysilane (III).

2. Procédé selon la revendication précédente, **caractérisé en ce que**, pour le diisocyanate (I), le radical Z est choisi parmi les radicaux (1) à (6) suivants : 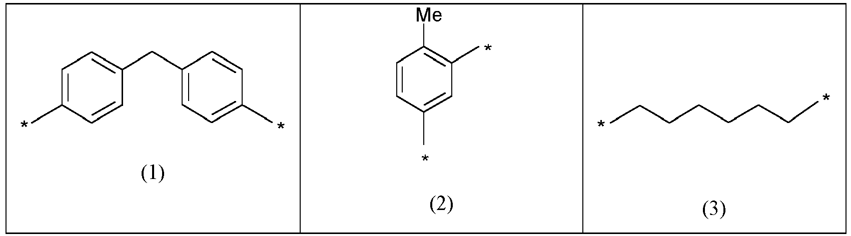 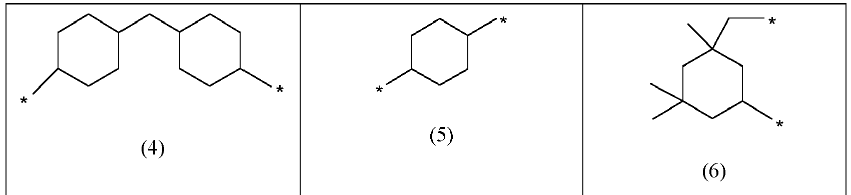 , préférablement, Z désigne le radical divalent isophorone (radical (6)).

3. Procédé selon l'une ou l'autre des revendications précédentes, **caractérisé en ce que**, pour le composé difonctionnel de formule (II) :
T désigne O ou NH ;
A désigne un radical hydrocarboné linéaire ou ramifié en C₂-C₅₀ éventuellement interrompu par un ou plusieurs atomes d'oxygène non adjacents ;
préférablement, A désigne un radical divalent choisi parmi :
(i) un radical divalent -[(CH₂)ₙ-O-]ₘ-(CH₂)_{n'}- avec n et n' désignant indépendamment un nombre entre 1 et 10, de préférence entre 1 et 5, m désignant un nombre compris entre 1 et 30, de préférence entre 1 et 20, plus particulièrement entre 1 et 10 ;
(ii) un radical alkylène, qui est en particulier non linéaire, en C₂-C₅₀, préférablement en C₂₀-C₅₀, en particulier en C₃₂-C₄₀, tel qu'un radical en C₃₆ ;
(iii) un radical divalent -[CH(Me)-CH₂-O-]ₓ-(CH₂)-CH(Me)- avec x désignant un nombre entre 2 et 70, préférablement entre 2 et 7 ;
(iv) un radical divalent -CH(Me)-CH₂-[O-CH₂-CH(Me)]_{x'}-[O-CH₂-CH₂]_{y'}-[O-CH₂-CH(Me)-]_{z'} avec y' désignant un nombre compris entre 2 et 50, préférablement 2 et 40 , x' + z' désignant un nombre entre 1 et 10, préférablement entre 1 et 7 ;
(v) un radical divalent -(CH₂)_{x'}-O-CH₂-CH₂-O-(CH₂)_{x"}- avec x" désignant un nombre entre 1 et 10, préférablement entre 1 et 5 .

4. Procédé selon l'une des revendications précédentes, **caractérisé en ce que**, pour le premier alcoxysilane (III) :
X₁ désigne -NRa-, de préférence -NRa- désigne -NH- ou -N-cyclohexyle ;
- R₁ désigne un radical méthyle ou éthyle ;
- R₂ et R₃, qui peuvent être identiques ou différents, sont choisis parmi :
- les radicaux méthoxy et éthoxy ;
- les radicaux méthyle et éthyle, préférablement méthyle ;
L₁ représente un radical hydrocarboné linéaire ou ramifié, saturé, en C₁-C_{10'} préférablement un radical hydrocarboné linéaire saturé en C₁-C₁₀ ; préférablement lorsque p = 1, L₁ représente un radical divalent saturé en C₁-C₈, préférablement un radical divalent choisi parmi -CH₂-CH₂- et -(CH₂)₆-.

5. Procédé selon l'une des revendications précédentes, **caractérisé en ce que** le premier alcoxysilane (III) est choisi parmi ceux de formule (IIIa) ci-dessous :
(R₁O)(R₂)(R₃)Si-CH₂-(NH-L₁)ₚ-NRa-H (IIIa)
dans laquelle :
p = 0 ou 1 ;
R₁ désigne un radical méthyle ou éthyle ; R₂ et R₃, qui peuvent être identiques ou différents, désignent un radical méthoxy, éthoxy, méthyle ou éthyle ;
lorsque p = 1, L₁ représente un radical divalent hydrocarboné saturé en C₁-C₈ ;
Ra désigne H ou un radical (cyclo)alkyle saturé ou insaturé en C₁-C₈, ou un radical phényle ; préférablement Ra désigne H ou un radical cyclohexyle.

6. Procédé selon l'une des revendications précédentes, **caractérisé en ce que** le premier alcoxysilane (III) est choisi parmi :
1-(diméthoxyméthylsilyl)méthanamine
1-(diéthoxyméthylsilyl)méthanamine
1-(triéthoxysilyl)méthanamine
1-(triméthoxysilyl)méthanamine
1-(triméthoxysilyl)méthanethiol
1-(diéthoxyméthylsilyl)méthanethiol
1-(triéthoxysilyl)méthanethiol
1-(triéthoxysilyl)méthanol
N-[(triéthoxysilyl)méthyl]benzènamine
N-[(triméthoxysilyl)méthyl]benzènamine
N-[(diéthoxyméthylsilyl)méthyl]cyclohexanamine
N-[(triéthoxysilyl)méthyl]cyclohexanamine
N-[(diméthoxyméthylsilyl)méthyl]-cyclohexanamine
N-(diéthoxyméthylsilyl)-N-méthylméthanamine
N-méthyl-1-(triméthoxysilyl)méthanamine
N-méthyl-1-(triéthoxysilyl)méthanamine
N-[(diméthoxy(méthyl)silyl)méthyl]benzènamine
N-[(triéthoxysilyl)méthyl]-1,6-hexanediamine
N-[(triméthoxysilyl)méthyl]-1,6-hexanediamine
N-[(diéthoxyméthylsilyl)méthyl]-1,6-hexanediamine
N-[(triméthoxysilyl)méthyl]-1,2-éthanediamine ,
préférablement parmi :
1-(diéthoxyméthylsilyl)méthanamine
1-(triéthoxysilyl)méthanamine
N-[(diéthoxyméthylsilyl)méthyl]cyclohexanamine
N-[(triéthoxysilyl)méthyl]cyclohexanamine
N-[(triéthoxysilyl)méthyl]-1,6-hexanediamine ,
et préférentiellement parmi :
N-[(triéthoxysilyl)méthyl]-1,6-hexanediamine
N-[(triéthoxysilyl)méthyl]-cyclohexanamine.

7. Procédé selon l'une des revendications précédentes, **caractérisé en ce que**, pour le deuxième alcoxysilane (IV) :
X₂ désigne -NRb- ou S, Rb désignant H ou un radical (cyclo)alkyle saturé ou insaturé en C₁-C₈, en particulier un radical méthyle, éthyle, butyle ou cyclohexyle, ou un radical aryle en C₆-C₁₀, en particulier phényle ; préférablement, X₂ désigne -NH- ou S ;
L₂ représente un radical hydrocarboné saturé, linéaire ou ramifié, en C₁-C₁₂, éventuellement interrompu par un groupe -NH- ; préférablement, lorsque q = 1, L₂ représente un radical saturé divalent en C₁-C₁₀, ou alors un radical divalent -(CH₂)ₙ-NH-(CH₂)ₘ avec n et m désignant des entiers tels que 2 ≤ n + m ≤ 4, et en particulier un radical divalent choisi parmi -CH₂-, -CH₂-CH₂-, -(CH₃)(CH₃)C-CH₂-, -(CH₂)₉- et -CH₂-NH-C₂H₄- ;
R'₁ désigne un radical méthyle ou éthyle ;
R'₂ et R'₃, qui peuvent être identiques ou différents, sont choisis parmi :
- des radicaux méthoxy ou éthoxy ;
- des radicaux méthyles ou éthyle, préférablement méthyle ;
R'₄ et R'₅ désignent H.

8. Procédé selon l'une des revendications précédentes, **caractérisé en ce que** le deuxième alcoxysilane (IV) est choisi parmi ceux de formule (IVa) ci-dessous :
(R'₁O)(R_{'2})(R₃)Si-CH₂-CH₂-(L₂)_{q}-NRb-H (IVa)
dans laquelle :
q = 0 ou 1 ;
Rb désignant H ou un radical (cyclo)alkyle saturé ou insaturé en C₁-C₈, tel qu'un radical méthyle, éthyle, butyle ou cyclohexyle, ou un radical phényle ;
R'₁ désigne un radical méthyle ou éthyle ; R'₂ et R'₃, qui peuvent être identiques ou différents, désignent un radical méthoxy, éthoxy, méthyle ou éthyle ;
L₂ désigne un radical hydrocarboné saturé, linéaire ou ramifié, en C₁-C₁₂, éventuellement interrompu par un groupe -NH- ; préférablement, lorsque q = 1, L₂ représente un radical saturé divalent en C₁-C₁₀, ou alors un radical divalent -(CH₂)ₙ-NH-(CH₂)ₘ avec n et m désignant des entiers tels que 2 ≤ n + m ≤ 4, et en particulier un radical divalent choisi parmi -CH₂-, -CH₂-CH₂-, -(CH₃)(CH₃)C-CH₂-, -(CH₂)₉- et -CH₂-NH- C₂H₄-.

9. Procédé selon l'une des revendications précédentes, **caractérisé en ce que** le deuxième alcoxysilane (IV) est choisi parmi :
3-diméthoxyméthylsilyl)-1-propanamine
3-(triméthoxysilyl)-1-propanamine
3-(triéthoxysilyl)1-propanamine
3-(diéthoxyméthoxysilyl)-1-propanamine
2-méthyl-3-(triméthoxysilyl)-1-propanamine
3-(triéthoxysilyl)-1-propanamine
3-(diéthoxyméthylsilyl)-1-propanamine
3-(méthyldipropoxysilyl)-1-propanamine
3-(diéthoxyéthylsilyl)-1-propanamine
3-(éthyldiméthoxysilyl)-1-propanamine
4-(triéthoxysilyl)-1-butanamine
4-(diméthoxyméthylsilyl)-1-butanamine
4-(triméthoxysilyl)-1-butanamine
2,2-diméthyl-4-(triméthoxysilyl)-1-butanamine
4-(diéthoxyméthylsilyl)-1-butanamine
4-(diméthoxyméthylsilyl)-2,2-diméthyl-1-butanamine
11-(triéthoxysilyl)-1-undécamine
11-(triméthoxysilyl)-1-undécamine
2-[(diméthoxyméthylsilyl)méthyl]-1,4-butanediamine
2-[(triméthoxysilyl)méthyl]-1,4-butanediamine
N-(3-(triméthoxysilyl)propyl)butylamine
N-éthyl-3(triméthoxysilyl)-1-propanamine
N-méthyl-3-(triméthoxysilyl)propylamine
N[3-triméthoxysilyl]propyl]cyclohexylamine
N[3-triméthoxysilyl]propyl]aniline
N[3-triméthoxysilyl]propyl]éthylènediamine
N[3-triéthoxysilyl]propyl]éthylènediamine
1-(triméthoxysilyl)-2-propanamine
2-(triméthoxysilyl)-éthanamine
2-(triéthoxysilyl)1-propanamine
2-(diméthoxyméthylsilyl)-éthanamine
2-(diéthoxyméthylsilyl)-1-propanamine
2-(diéthoxyméthylsilyl)-éthanamine
2-(triéthoxysilyl)-éthanamine
4-(triméthoxysilyl)-1-butanol
3-triméthoxysilyl)-1-propanol
11-(triméthoxysilyl)-1-undécanethiol
4-(triméthoxysilyl)-2-butanethiol
2-(triéthoxysilyl)-éthanethiol
3-(triéthoxysilyl)1-propanethiol
2-(triméthoxysilyl)-éthanethiol
3-(triméthoxysilyl)-1-propanethiol
3-(diméthoxyméthylsilyl)-1-propanethiol
N-[3-(triméthoxysilyl)propyl]acétamide
préférablement parmi :
3-(triéthoxysilyl)-1-propanamine
3-(diéthoxyméthoxysilyl)-1-propanamine
3-(triéthoxysilyl)-1-propanamine
3-(diéthoxyméthylsilyl)-1-propanamine
3-(diéthoxyéthylsilyl)-1-propanamine
4-(triéthoxysilyl)-1-butanamine
4-(diéthoxyméthylsilyl)-1-butanamine
11-(triéthoxysilyl)-1-undécamine
N-[3-triéthoxysilyl]propyl]iéthylènediamine
2-(triéthoxysilyl)-éthanethiol
3-(triéthoxysilyl)1-propanethiol,
et préférentiellement choisi parmi (3-aminopropyl)triéthoxysilane et 3-(triéthoxysilyl)1-propanethiol.

10. Procédé selon l'une des revendications précédentes, **caractérisé en ce que** le mélange des premier et deuxième alcoxysilanes (III) et (IV) utilisés comprend de 5 à 95 % en moles d'alcoxysilane (III), par rapport au nombre de moles total d'alcoxysilanes (III) et (IV), préférablement de 20 à 80 % en moles, préférentiellement de 30 à 70 % en moles, et plus préférentiellement de 50 à 70 % en moles.

11. Procédé selon l'une des revendications précédentes, **caractérisé en ce que** les réactifs sont utilisés selon les équivalents molaires suivants :
diisocyanate (I) : 2 équivalents
composé difonctionnel (II) : 1 équivalent
premier alcoxysilane (III) : u équivalent
deuxième alcoxysilane (IV) : v équivalent
avec u + y = 2, u et v n'étant pas zéro.

12. Procédé selon l'une des revendications précédentes, **caractérisé en ce que** la première étape est mise en œuvre en présence d'un catalyseur, en particulier un catalyseur organique à base d'étain, tel que le 2-éthylhexanoate d'étain, le dilaurate de dibutylétain, le dilaurate de dioctylétain, le tris(2-éthylhexanoate) de butylétain, le diacétate de dibutylétain ou le diacétate de dioctylétain, et de préférence le 2-éthylhexanoate d'étain.

13. Procédé selon l'une des revendications précédentes, **caractérisé en ce que** la première étape est mise en œuvre dans un solvant aprotique, préférablement choisi parmi le méthyltétrahydrofuranne, le tétrahydrofuranne et le toluène, à une température comprise entre 40 °C et 120 °C, préférablement entre 50 °C et 70 °C.

14. Procédé selon l'une des revendications précédentes, **caractérisé en ce que** la deuxième étape est mise en œuvre à une température comprise entre 20 °C et 60 °C.

15. Procédé selon l'une des revendications précédentes, **caractérisé en ce que** la deuxième étape est suivie d'une étape d'échange de solvant par élimination du solvant aprotique et ajout d'un solvant véhicule, tel qu'un solvant de type alcool, notamment un C₂C₂₂-alcool, préférablement choisi parmi l'éthanol, l'isopropanol, le propanol, le t-butanol, le sec-butanol et le 2-octyldodécanol.

16. Procédé selon l'une des revendications précédentes, **caractérisé en ce que** le polymère obtenu comprenant un groupe alcoxysilane est véhiculé dans un solvant véhicule, en particulier un solvant de type alcool tel que défini dans la revendication précédente.

17. Produit qui est un polymère comprenant un groupe alcoxysilane (Pf), qui est obtenu par le procédé de préparation selon l'une quelconque des revendications précédentes.

18. Mélange de composés C1, C2 et C3 :
(R₁O)(R₂)(R₃)Si-CH_{z}-(NH-L₁)ₚ-X₁-CO-NH-Z-NH-CO-T-A-T-CO-NH-Z-NH-CO-X₁-(L₁-NH-)ₚ-CH_{z}-Si(R₁O)(R₂)(R₃) (C1)
(R₁O)(R₂)(R₃)Si-CH₂-(NH-L₁)ₚ-X₁-CO-NH-Z-NH-CO-T-A-T-CO-NH-Z-NH-CO-X₂-(L₂)_{q}-CH(R₅)-CH(R₄)-........ .......Si (R'₁O)(R'₂)(R'₃) (C2)
(R'₁O)(R'₂)(R'₃)Si-CH(R₄)-CH(R₅)-(L₂)_{q}-X₂-CO-NH-Z-NH-CO-T-A-T-CO-NH-Z-NH-CO-X₂-(L_{z})_{q}-CH(R₅)-CH(R₄)-...... ........-Si (R'₁O)(R'₂)(R'₃) (C3)
dans lesquels Z, T, A, R₁, R₂, R₃, R'₁, R'₂, R'₃, R₄, R₅, L₁, L₂, X₁, X₂, p et q ont les significations définies dans les revendications 1 à 4 et 6 et 7.

19. Composés de formule C2 :
(R₁O)(R₂)(R₃)Si-CH₂-(NH-L₁)ₚ-X₁-CO-NH-Z-NH-CO-T-A-T-CO-NH-Z-N-CO-X₂-(L₂)_{q}-CH(R₃)-CH(R₄)-..... .......Si (R'₁O)(R'₂)(R'₃), (C2)
dans laquelle Z, R₁, R₂, R₃, R'₁, R'₂, R'₃, R₄, R₅, L₁, L₂, X₁, X₂, p et q ont les significations définies dans les revendications 1 à 4 et 6 et 7, et
T désigne O ou NH ;
A désigne un radical hydrocarboné linéaire ou ramifié en C₂C₅₀ éventuellement interrompu par un ou plusieurs atomes d'oxygène non adjacents.

20. Composition anhydre comprenant, dans un milieu physiologiquement acceptable, un produit ou (des) composé(s) tel(s) que défini(s) selon l'une des revendications 17 à 19.

21. Composition selon la revendication précédente, **caractérisée en ce que** le produit ou les composés sont présents en une teneur dans la plage de 0,1 % à 60 % en poids, par rapport au poids total de la composition, de préférence dans la plage de 0,1 % à 50 % en poids, préférentiellement dans la plage de 0,5 % à 45 % en poids.

22. Composition selon la revendication 20 ou 21, **caractérisée en ce qu'**elle comprend au moins un solvant organique volatil.

23. Procédé cosmétique pour le soin ou le maquillage de matières kératiniques, en particulier des ongles ou des cheveux ou de la peau, comprenant l'application aux matières kératiniques, en particulier aux ongles ou aux cheveux ou à la peau, d'une composition selon l'une ou l'autre des revendications 21 et 22.
